# EUROPÄISCHE PATENTSCHRIFT

(11) **EP 0 339 208 B1**
(45) Veröffentlichungstag und Bekanntmachung des Hinweises auf die Patenterteilung: **07.02.1996**
(21) Anmeldenummer: 89103651.9
(22) Anmeldetag: 02.03.1989
(51) Int. Cl.: C07D 403/12, C07D 401/14, A61K 31/50

(54) **6-Oxo-pyridazinderivate, Verfahren zu ihrer Herstellung und diese Verbindungen enthaltende Arzneimittel**
6-Oxopyridazine derivatives, process for their preparation and medicaments containing these compounds
Dérivés de 6-oxopyridazine, procédé pour leur préparation et médicaments contenant ces composés

(30) Priorität: 26.04.1988 DE 3814057
(43) Veröffentlichungstag der Anmeldung: 02.11.1989
(73) Patentinhaber: HEUMANN PHARMA GMBH & CO, D-90478 Nürnberg (DE)
(72) Erfinder: Mörsdorf, Peter, Dr. Dipl-Chem., D-8506 Langenzenn (DE); Herter, Rolf, Dr. Dipl.-Chem., D-8540 Schwabach (DE); Engler, Heidrun, Dr., D-8501 Cadolzburg (DE); Pfahlert, Volker, Dr., D-2055 Daffendorf (DE); Weidner, Reinhold, Dr. Dipl.-Chem., D-8500 Nürnberg (DE); Ahrens, Kurt-Henning, Dr., D-8500 Nürnberg (DE)
(74) Vertreter: Kraus, Walter, Dr.

(56) Entgegenhaltungen:
- EP-A- 0 208 518
- EP-A- 0 252 422
- EP-A- 0 262 448
- EP-A- 0 304 534
- WO-A-87/05016
- DE-A- 2 207 517
- DE-A- 2 837 161
- CHEMICAL ABSTRACTS, Band 103, Nr. 19, 11. November 1985, Seite 708, Zusammenfassung Nr. 160462a, Columbus, Ohio, USA; I. SIRCAR et al.: "Cardiotonic agents. 2. Synthesis and structure-activity relationships of 4,5-dihydro-6-(4-(H-imidazol-1-yl)phenyl)-3(2H)-pyridazinones: a new class of positive inotropic agents"; & J. Med. Chem. 1985, 28(10), 1405-13
- JOURNAL OF MEDICINAL CHEMISTRY, Band 28, 1985, Seiten 1414-1422; G.J. DURANT et al.: "The Histamine H2 Receptor Agonist Impromidine: Synthesis and Structure-Activity Considerations"
- Drugs of the Future, Vol. 14, no. 12, 1989, pp. 1161-1162

## Beschreibung

Digitalisglykoside, wie Digoxin und Digitoxin, sind seit nunmehr 200 Jahren in der Therapie der Herzinsuffizienz eingeführt. Daneben sind die Sympathomimetika die einzige therapeutische Alternative. Beide Substanzklassen weisen jedoch eine Reihe von Nachteilen, wie geringe therapeutische Breite, Tachyphylaxie oder fehlende orale Verfügbarkeit, auf.

Außerdem ist die Wirksamkeit der Sympathomimetika bei gewissen Krankheitsbildern aus prinzipiellen Gründen eingeschränkt, da es zum Beispiel beim Herzinfarkt oder der kongestiven Cardiomyopathie zu einer ausgeprägten Schädigung des β-adrenergen Systems mit einer Down-Regulation der Rezeptoren kommt.

Histamin-H₂-Agonisten, wie zum Beispiel Impromidin (G.J. Durant et al., J.Med.Chem. 28, 1414 (1985)), sind eine neue Gruppe von cardiotonen Substanzen, die aufgrund ihres anderen Wirkungsmechanismus für die Behandlung der oben genannten Erkrankungen eine interessante Alternative darstellen. Es ist außerdem bekannt, daß Pyridazinonderivate, wie zum Beispiel Pimobendan (INN) (DE-OS 28 37 161, US-PS 4 361 563), und Impromidin sich in ihrer positiv inotropen Wirkung potenzieren.

Weiterhin sind aus der EP-A-0 208 518 6-Phenyl-pyridazinyl-verbindungen der allgemeinen Formel 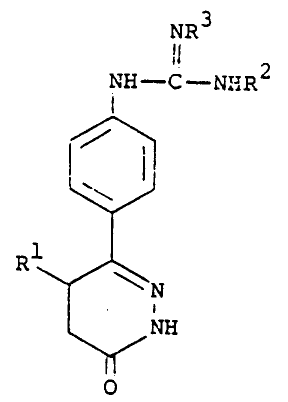
worin R¹, R² und R³ die in dieser Druckschrift angegebenen Bedeutungen haben. Nach den Angaben dieser Druckschrift handelt es sich bei den darin beschriebenen Verbindungen um positiv inotrope Verbindungen. Hinweise auf eine PDE-hemmende Wirkung dieser Dihydropyridazinonderivate finden sich aber in der EP-A-0 208 518 nicht.

In der EP-A-0 262 448 werden neue Imidazolylguanidinderivate beschrieben, die aufgrund ihrer agonistischen Wirkung auf Histamin-H₂-Rezeptoren sowie zum Teil wegen ihrer zusätzlichen H₁-antagonistischen Rezeptoraktivität bei Erkrankungen des Herzens, bei bestimmten Formen der Hypertonie sowie bei arteriellen Verschlußkrankheiten eingesetzt werden können. Diese Imidazolylguanidinderivate sind durch die allgemeine Formel 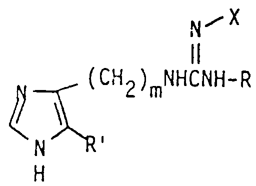
charakterisiert, worin R, R' und X die dort angegebenen Bedeutungen besitzen. Auch in dieser Druckschrift finden sich keine Hinweise auf eine PDE-hemmende Wirkung der dort beschriebenen Imidazolylguanidinderivate.

Gegenstand der EP-A-0 304 534, die unter Artikel 54(3) EPÜ fällt, sind Dihydropyridazinon-Derivate der allgemeinen Formel 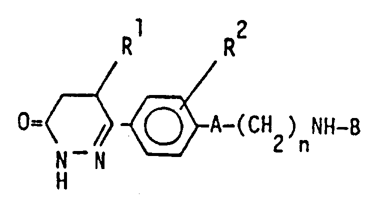
worin R¹, R², A und B die dort angegebenen Bedeutungen besitzen. Auch für diese Verbindungen werden nur positiv inotrope Wirkungen genannt.

Demgegenüber liegt der Erfindung die Aufgabe zugrunde, neue positiv inotrope Verbindungen herzustellen, die eine höhere und selektivere inotropiesteigernde Wirksamkeit aufweisen.

Gegenstand der Erfindung sind daher 6-Oxo-pyridazinderivate der allgemeinen Formel (I) 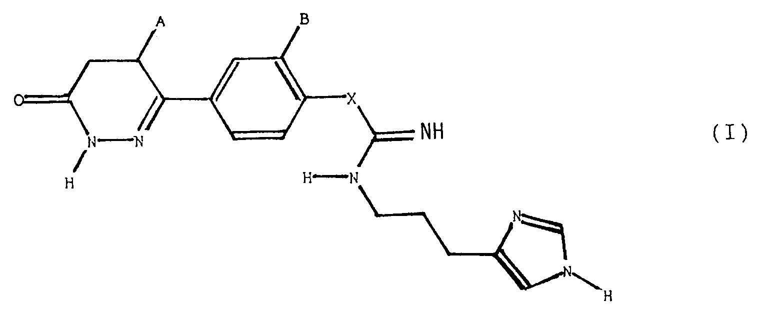
in der A für ein Wasserstoffatom, eine C₁-C₃-Alkylgruppe oder eine Hydroxymethylgruppe steht,
B ein Wasserstoffatom, ein Halogenatom, eine Cyanogruppe oder eine Nitrogruppe ist,
X die Gruppe 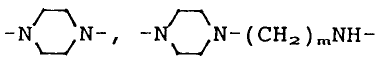
oder 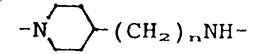
bedeutet, worin m den Wert 2, 3, 4, 5 oder 6 und n den Wert 0, 1, 2, 3 oder 4 haben,
sowie die physiologisch annehmbaren Salze davon.

In der allgemeinen Formel I bedeutet A ein Wasserstoffatom, eine C₁-C₃-Alkylgruppe oder eine Hydroxymethylgruppe.

Beispiele für die C₁-C₃-Alkylgruppe sind die Methyl-, Ethyl-, n-Propyl- und die Isopropylgruppe, wobei die Methylgruppe bevorzugt wird. B bedeutet ein Wasserstoffatom, ein Halogenatom, beispielsweise ein Fluor-, Chlor- oder Bromatom, eine Cyanogruppe oder eine Nitrogruppe. Von den Halogenatomen werden das Fluor- und das Chloratom bevorzugt. Besonders bevorzugt werden Verbindungen, bei denen B für eine Nitrogruppe steht.

X bedeutet die Gruppe 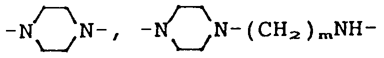
oder 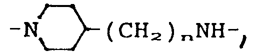
worin m den Wert 2, 3, 4, 5 oder 6 hat, wobei der Wert 2, 3 oder 4 bevorzugt wird. n hat den Wert 0, 1, 2, 3 oder 4.

Eine bevorzugte Gruppe von erfindungsgemäßen Verbindungen ist dadurch gekennzeichnet, daß X die Gruppe 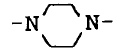
ist.

Eine weitere bevorzugte Gruppe von erfindungsgemäßen Verbindungen ist dadurch gekennzeichnet, daß X die Gruppe 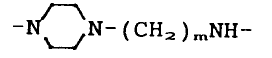
bedeutet.

Eine weitere Gruppe von bevorzugten Verbindungen ist dadurch gekennzeichnet, daß X die Gruppe 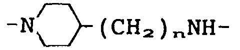
bedeutet, worin n den Wert 0, 1, 2, 3 oder 4 hat.

Bevorzugte Einzelverbindungen sind die folgenden Verbindungen:
6-[4-[4-[3-(1H-Imidazol-4-yl)-propylamino-iminomethylen]-piperazin-1-yl]-3-nitro-phenyl]-4,5-dihydro-5-methyl-3(2H)-pyridazinon,
N¹-[3-(1H-Imidazol-4-yl)propyl]-N³-[3-[4-(4-(4-methyl-6-oxo-1,4,5,6-tetrahydropyridazin-3-yl)-2-nitro-phenyl]-piperazin-1-yl]propyl]-guanidin,
N¹-[3-(1H-Imidazol-4-yl)propyl]-N³-[2-[4-(4-methyl-6-oxo-1,4,5,6-tetrahydropyridazin-3-yl)-2-nitro-anilino]-ethyl]-guanidin.

Die erfindungsgemäßen Verbindungen sowie die physiologisch annehmbaren Salze davon können durch ein Verfahren hergestellt werden, das dadurch gekennzeichnet ist, daß man
a) eine Verbindung der allgemeinen Formel (II) 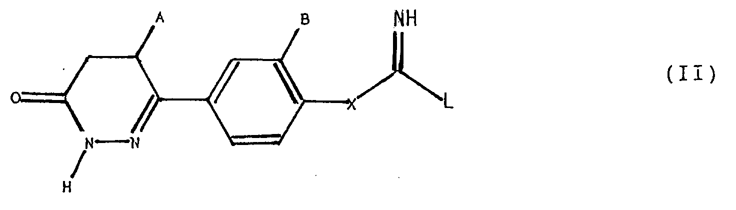 in der A, B und X wie in Anspruch 1 definiert sind und L für eine C₁-C₄-Alkylthio-, eine Phenylthio-, eine C₁-C₄-Alkoxy- oder eine Phenoxygruppe steht,
   mit einer Verbindung der Formel (III) 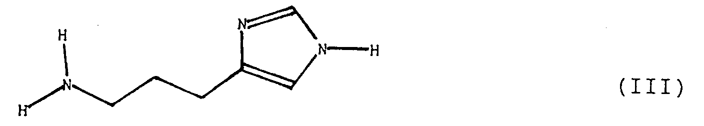 zu einer Verbindung der allgemeinen Formel (I) umsetzt oder
b) eine Verbindung der allgemeinen Formel (IV) 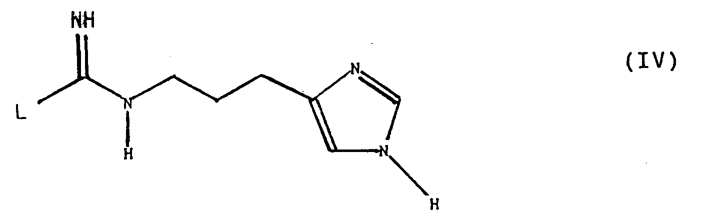 in der L die oben angegebene Bedeutung besitzt,
   mit einer Verbindung der allgemeinen Formel (V) 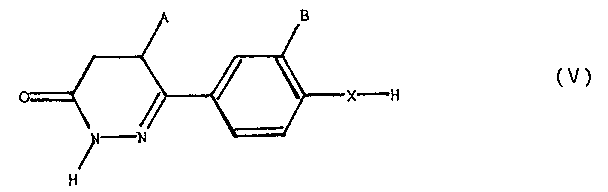 in der A, B und X wie oben definiert sind,
   zu einer Verbindung der allgemeinen Formel (I) umsetzt oder
c) eine Verbindung der allgemeinen Formel (Ia) 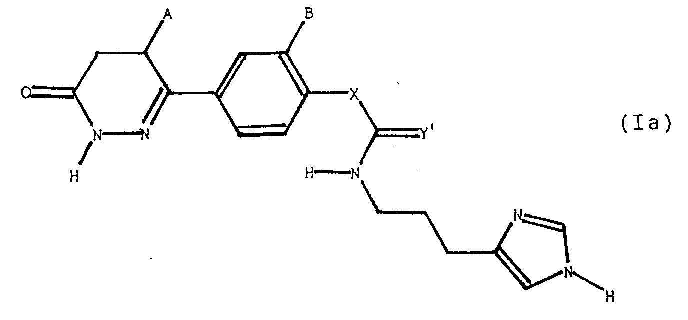 in der A, B und X die oben angegebenen Bedeutungen haben und Y' für eine der Gruppen =N-CN, 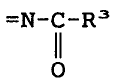 oder 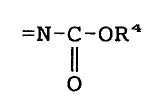 steht, worin R³ für eine gerad- oder verzweigtkettige C₁-C₆-Alkylgruppe oder eine gegebenenfalls mit einem oder mehreren Halogenatomen, C₁-C₃-Alkylgruppen oder C₁-C₃-Alkoxygruppen substituierte Phenyl- oder Naphthylgruppe steht und R⁴ für eine gegebenenfalls mit einem oder mehreren Halogenatomen, C₁-C₃-Alkoxygruppen oder Phenylresten substituierte gerad- oder verzweigtkettige C₁-C₄-Alkylgruppe steht,
   sauer oder basisch zu einer Verbindung der allgemeinen Formel (I) hydrolysiert und gegebenenfalls die nach den Verfahrensvarianten a) bis c) erhaltene Verbindung der allgemeinen Formel (I) in an sich bekannter Weise in ihr physiologisch annehmbares Salz umwandelt.

Die Verbindungen der allgemeinen Formeln II und IV werden vorzugsweise im Verhältnis 1:1 bis 0,8:1, besonders bevorzugt in äquimolaren Mengen, bezogen auf die Verbindungen der allgemeinen Formeln III bzw. V, eingesetzt. Die Umsetzungen werden in einem polaren Lösungsmittel, wie Acetonitril, Pyridin, Dimethylformamid, oder Alkohol, vorzugsweise sekundärem oder tertiärem Alkohol, wie zum Beispiel Isopropanol oder Gemischen davon, und bei Temperaturen von 20°C bis zur Rückflußtemperatur des verwendeten Lösungsmittels durchgeführt.

Im Falle der Variante (c) erfolgt die saure Hydrolyse beispielsweise in einer wäßrigen Mineralsäure, wie Chlor- oder Bromwasserstoffsäure oder Schwefelsäure, und bei Temperaturen von 20 bis 100°C, vorzugsweise 30 bis 80°C. Gegebenenfalls kann es aber auch günstiger sein, nicht-wässrige Reaktionsbedingungen anzuwenden. Solche schonenderen Methoden sind z. B. die Solvolyse mit Trifluoressigsäure in einem Chlorkohlenwasserstoff, wie Dichlormethan oder Chloroform, oder die Umsetzung mit Bromwasserstoff in Eisessig.

Die basische Hydrolyse erfolgt in einer verdünnten Lösung von Alkali- oder Erdalkalicarbonaten bzw. Alkali- oder Erdalkalihydroxiden in Wasser, niedrigen Alkoholen oder Gemischen von beiden und bei Temperaturen von Raumtemperatur bis zur Rückflußtemperatur des verwendeten Lösungsmittels.

Die nach den einzelnen Verfahrensvarianten erhaltenen Verbindungen werden in üblicher Weise isoliert und gereinigt, beispielsweise durch Umkristallisation, chromatographische Arbeitsweisen usw. Die bei den einzelnen Verfahrensvarianten erhaltenen Verbindungen können gegebenenfalls in ihr physiologisch annehmbares Salz umgewandelt werden. Diese Salze können zum Beispiel mit Mineralsäuren, wie Chlor-, Brom-, Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc. gebildet werden.
Die erfindungsgemäßen Verbindungen der allgemeinen Formel I können sowohl in einer Reihe von tautomeren Formen als auch in mehreren stereoisomeren Formen vorliegen. Die Erfindung umfaßt daher neben den Salzen und Hydraten der oben beschriebenen Verbindungen der allgemeinen Formel I auch alle tautomeren und stereoisomeren Formen.
Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung eines oder mehrerer pharmazeutisch annehmbarer Träger oder Verdünnungsmitteln hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen. Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.
Alternativ kann der Wirkstoff auch in Pulverform zur Rekonstitution mit einem geeigneten Träger, zum Beispiel sterilem, pyrogenfreiem Wasser, vor dem Gebrauch vorliegen.
Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.
Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, in denen mit weniger als der oben genannten Mindestmenge ausgekommen werden kann, während in anderen Fällen die genannte obere Grenze überschritten werden muß.

Die erfindungsgemäßen 6-Oxo-pyridazinderivate der allgemeinen Formel I zeigen ausgeprägte cardiovaskuläre, insbesondere cardiotone Wirkungen und eignen sich daher zur Behandlung und Vorbeugung von Erkrankungen des Herzens und des Kreislaufs.

So zeigen sie eine ausgezeichnete positiv inotrope Wirkung in einer Reihe von in vitro- und in vivo- Standardmodellen, z. B. im isolierten, perfundierten Langendorff-Herzen und am narkotisierten Meerschweinchen nach intravenöser Applikation.

### 1.) Positiv inotrope Wirkung am isolierten, perfundierten Langendorff-Herzen (Meerschweinchen)

a) Methode
Zur Bestimmung der hämodynamischen Effekte der erfindungsgemäßen Verbindungen an isolierten, perfundierten Meerschweinchenherzen wurde die Anordnung von Langendorff nach P.R.Beckett (J. Pharm. Pharmacol 22,818 (1970)) und R. M. Abel und R. L. Reis (Circ. Res. 27, 961 (1970)) modifiziert. Die spontan schlagenden Meerschweinchenherzen sind linksventrikulär katheterisiert und werden mit Lösungen der Prüfsubstanzen in physiologischer Kochsalzlösung/Ethanol (9:1) in Konzentrationen von 1o⁻⁴ bis 10⁻⁸ mol/l mit einem konstanten Perfusionsdruck von 60 mm Hg perfundiert.
b) Meßwerte

| Beispiel Nr. | Konzentration (mol/l) | Zunahme der Kontraktilität LV dp/dt gegenüber den Ausgangswerten |
|---|---|---|
| 1 | 10⁻⁶ | + 90 % |
| | 10⁻⁵ | + 100 % |
| 3 | 10⁻⁶ | + 50 % |
| | 10⁻⁵ | + 100 % |
| 5 | 10⁻⁵ | + 200 % |
| 6 | 10⁻⁷ | + 67 % |
| | 10⁻⁶ | + 220 % |

### 2. Hämodynamische Charakterisierung am narkotisierten Meerschweinchen (i. v.- Applikation)

a) Methode
Die Tiere werden mit Urethan (1,5 g/kg) narkotisiert. Zur volumenkontrollierten Beatmung wird die Trachea kanüliert. Danach erfolgt die operative Freilegung beider Karotiden; über die rechte Karotis wird ein Tip-Katheter (3F) eingeführt, der unter fortlaufender Druckregistrierung dann über die Aorta ascendens in den linken Ventrikel vorgeführt wird. Die erfolgreiche Passage der Aortenklappen wird hierbei durch die typische linksventrikuläre Druckkurve erkannt. Über die linke Karotis wird zur Thermodilution ein Thermistorfühler (3F, F. Edwards) in den Aortenbogen vorgeschoben. Der Thermistorfühler hat gleichzeitig ein Lumen zur arteriellen Blutdruckregistrierung. Zur Applikation des Kälteinjektats (0,2 ml 0,9 % NaCl, 15°C) wird durch die rechte Vena jugularis ein Katheter vor den rechten Vorhof plaziert. Alle Substanzen sind in physiologischer Kochsalzlösung gelöst und werden über die linke Vena jugularis infundiert (Infusionsvolumen 0,02 ml/min); die Applikation erfolgt nach hämodynamischer Stabilisierung und unter β-Blockade (Metoprolol 2 mg/kg i.m.). Alle Kreislaufparameter werden kontinuierlich auf einem Direktschreiber registriert. Die Kontraktilität (dp/dt) wird über die Volumenkurve berechnet.
b) Meßwerte

| Beispiel Nr. | Dosis µg/kg/min | Maximale Zunahme der Kontraktilität LV dp/dt |
|---|---|---|
| 1 | 5 | + 100 % |
| 3 | 10 | + 90 % |
| 6 | 5 | + 200 % |

Die folgenden Beispiele erläutern die Erfindung. Es wird jedoch angemerkt, daß die Beispiele 1, 4, 9 und 18-22 sich nicht auf in den Ansprüchen definierte Verbindungen beziehen. Die Zwischenprodukte wurden routinemäßig auf die Reinheit durch Dünnschichtchromatographie überprüft, wobei für den Nachweis UV-Licht sowie Sprühreagentien, wie Echtblausalz B/Natronlauge, verwendet wurden.

Die präparative Chromatographie wurde unter Verwendung von Kieselgel (Merck, Art. Nr. 7734 und 7749) durchgeführt.

Die Dünnschichtchromatographie wurde an Kieselgel-Folien Polygram SIL G/UV₂₅₄ (Machery-Nagel) durchgeführt.

Für die Elutionsmittel werden folgende Abkürzungen verwendet:

| | | |
|---|---|---|
| A | Dichlormethan/Methanol | 95:5 |
| B | Dichlormethan/Methanol/konz. Ammoniak | 90:7:3 |
| C | Dichlormethan/Methanol/konz. Ammoniak | 85:13:2 |
| D | Ethylacetat/Puffer* | 50:50 |
| E | Ethylacetat/Puffer* | 60:40 |
| F | Ethylacetat/Methanol/konz. Ammoniak | 80:18:2 |
| G | Ethylacetat/Puffer | 70:30 |
| H | Dichlormethan/Methanol | 90:10 |

| | | |
|---|---|---|
| * Puffer:Methanol/konz. Ammoniak gesättigt mit Ammoniumchlorid 95:5 | | |

### Beispiel 1

### N¹-[3-(1H-Imidazol-4-yl)propyl]-N³-[2-[4-(4-methyl-6-oxo-1,4,5,6-tetrahydropyridazin-3-yl)-2-nitro-anilino]ethyl]-guanidin

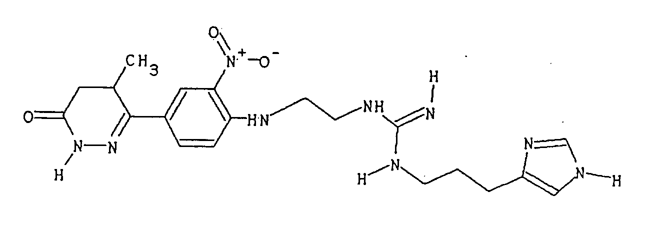

a) N¹-Benzoyl-N²-[2-[4-(4-methyl-6-oxo-1,4,5,6-tetrahydropyridazin-3-yl)-2-nitro-anilino]ethyl]-thioharnstoff
   Eine Suspension von 5,0 g (17,1 mmol) N-[4-(4-Methyl-6-oxo-1,4,5,6-tetrahydropyridazin-3-yl)-2-nitro-phenyl]-ethylendiamin in 50 ml Acetonitril wird mit 2,8 g (17,1 mmol) Benzoylisothiocyanat versetzt und eine Stunde bei Raumtemperatur gerührt. Der ausgefallene Feststoff wird abgesaugt, mit etwas Acetonitril gewaschen und mit 100 ml Ethanol aufgekocht. Nach Abkühlen und Absaugen erhält man 6,4 g (82%) eines orangegelben Feststoffs vom Schmp. 177°C.
   C₂₁H₂₂N₆O₄S (454,50)
   Rf = 0,73 (Laufmittel H)
b) N-[2-[4-(4-Methyl-6-oxo-1,4,5,6-tetrahydropyridazin-3-yl)-2-nitro-anilino]ethyl]-thioharnstoff
   2,00 g (4,4 mmol) N¹-Benzoyl-N²-[2-[4-(4-methyl-6-oxo-1,4,5,6-tetrahydropyridazin-3-yl)-2-nitro-anilino]ethyl]-thioharnstoff werden mit 1,20 g (8,7 mmol) Kaliumcarbonat in 75 ml Methanol und 15 ml Wasser eine Stunde gekocht. Der ausgefallene Feststoff wird abgesaugt und aus Methanol umkristallisiert. Man erhält 1,37 g (89%) eines orangefarbenen Feststoffs vom Schmp. 231-233°C.
   C₁₄H₁₈N₆O₃S (350,39)
   Rf = 0,43 (Laufmittel H)
c) N-[2-[4-(4-Methyl-6-oxo-1,4,5,6-tetrahydropyridazin-3-yl)-2-nitro-anilino]ethyl]-S-methyl-isothiuroniumjodid.
   1,35 g (3,85 mmol) des Thioharnstoffs aus Stufe b) werden mit 0,26 ml (4,2 mmol) Methyljodid 2,5 Stunden in 20 ml Dimethylformamid bei Raumtemperatur gerührt. Die Lösung wird filtriert und das Filtrat bei 70°C i. Vak. eingedampft. Der Rückstand ergibt nach Kristallisation mit 20 ml Ethanol 1,33 g (70%) orangefarbene Kristalle vom Schmp. 145-147°C.
   C₁₅H₂₁JN₆O₃S (492,33)
   Rf = 0,18 (Laufmittel H)
d) N¹-[3-(1H-Imidazol-4-yl)propyl]-N³-[2-[4-(4-methyl-6-oxo-1,4,5,6-tetrahydropyridazin-3-yl)-2-nitro-anilino]ethyl]-guanidin
   1,00 g (2,03 mmol) N-[2-[4-(4-Methyl-6-oxo-1,4,5,6-tetrahydropyridazin-3-yl)-2-nitro-anilino]ethyl]-S-methyl-isothiuroniumjodid und 0,28 g (2,23 mmol) 3-(1H-Imidazol-4-yl)-propylamin werden in 20 ml Pyridin 3 Stunden gekocht. Nach Abkühlen wird die rote Lösung i. Vak. eingedampft und der Rückstand an Kieselgel mit Ethylacetat/Methanol/konz. Ammoniak gesättigt mit Ammoniumchlorid (50:47,5:2,5) als Laufmittel chromatographiert. Die Hauptfraktion wird i. Vak. eingedampft, der Rückstand in 10 ml gesättigter Kaliumcarbonat-Lösung aufgenommen und die wässrige Phase mit 3x10 ml Isopropanol extrahiert. Die organischen Phasen ergeben nach Trocknen, Filtrieren und Eindampfen i. Vak. einen orangegelben Schaum, der mit Ethanol kristallisiert wird. Man erhält 0,40 g (45%) eines unscharf schmelzenden Feststoffs.
   C₂₀H₂₇N₉O₃ (441,49)
   Rf = 0,38 (Laufmittel D)
   - ¹H-NMR-Daten (DMSO-d₆, TMS als interner Standard): δ = 1,05 (d) 3H
   1,72 (quin) 2H
   2,15-2,80 (m) 4H
   3,10 (t) 2H
   3,2 -3,6 (m) 5H
   6,75 (s) 1H
   7,23 (d) 1H
   7,50 (s) 1H
   7,95 (dd) 1H
   8,38 (d) 1H
   8,6 (breit) 6H,
   austauschbar mit
   D₂O
   ppm

### Beispiel 2

### 6-[4-[4-[3-(1H-Imidazol-4-yl)propylamino-iminomethylen]piperazin-1-yl]phenyl]-4,5-dihydro-5-methyl-3(2H)-pyridazinon

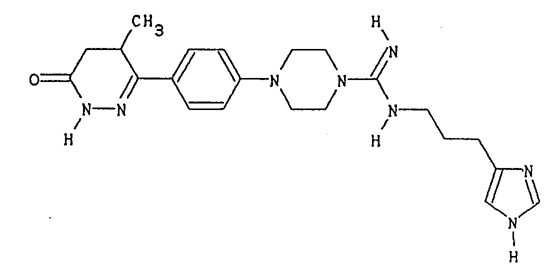

2,00 g (4,2 mmol) 6-[4-[4-(Methylthio-iminomethylen)piperazin-1-yl]phenyl]-4,5-dihydro-5-methyl-3(2H)-pyridazinon-hydrojodid und 0,58 g (4,7 mmol) 3-(1H-Imidazol-4-yl)-propylamin werden in 50 ml Acetonitril 3 Stunden unter Rückfluß gekocht. Das nach Eindampfen der Lösung i. Vak. erhaltene Öl wird mit Ethylacetat/Methanol/konz. Ammoniak gesättigt mit Ammoniumchlorid (70:28,5:1,5) als Laufmittel an Kieselgel chromatographiert. Die Hauptfraktion wird im Vakuum eingedampft, der Rückstand in 10 ml gesättigter Kaliumcarbonat-Lösung aufgenommen und mit 2 x 10 ml Isopropanol extrahiert. Nach Trocknen und Einengen der organ. Phase verbleibt ein amorpher Feststoff, der beim Verreiben mit wenig Isopropanol als Isopropanolat kristallisiert.
C₂₂H₃₀N₈OxC₃H₈O (482,61)
Rf = 0,55 (Laufmittel D)
- ¹H-NMR-Daten (DMSO-d₆,TMS als interner Standard): δ= 1,02-1,14 (2d) 9H
1,76 (quin) 2H
2,4-3,9 (m) 16H
6,70 (s) 1H
6,98 (d) 2H
7,49 (s) 1H
7,63 (d) 2H
10,85 (breit) 1 H
austauschbar mit D₂O

### Beispiel 3

### 6-[4-[4-[3-(1H-Imidazol-4-yl)propylamino-iminomethylen] piperazin-1-yl]-3-nitro-phenyl]-4,5-dihydro-5-methyl-3(2H)-pyridazinon

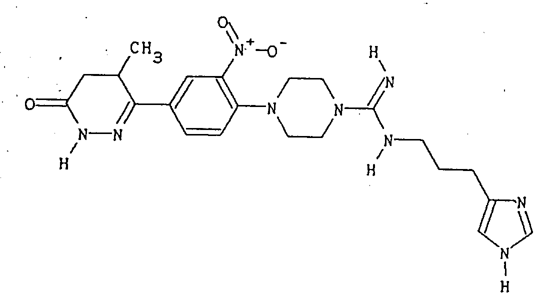

a) 6-[4-[4-(Benzoylamino-thiocarbonyl)piperazin-1-yl]-3-nitro-phenyl]-4,5-dihydro-5-methyl-3(2H)-pyridazinon
   52,4 g (0,165 mol) 6-[4-(1-Piperazinyl)-3-nitro-phenyl]-4,5-dihydro-5-methyl-3(2H)-pyridazinon in 1200 ml 1,4-Dioxan werden bei RT mit 27,0 g (0,165 mol) Benzoylisothiocyanat versetzt. Man rührt 2 h bei RT, konzentriert das Reaktionsgemisch auf 700 ml im Vakuum und läßt kristallisieren. Nach dem Absaugen erhält man 76,05 g (96% d. Th.) orangegelber Kristalle, die bei 174,5-177°C schmelzen.
   C₂₃H₂₄N₆O₄S (480,55)
   Rf = 0,58 (Laufmittel B)
b) 6-[4-[4-(Amino-thiocarbonyl)piperazin-1-yl]-3-nitro-phenyl]-4,5-dihydro-5-methyl-3(2H)-pyridazinon
   66,6 g (0,139 mol) der wie unter a) synthetisierten Verbindung werden in 1200 ml Methanol und 150 ml Wasser in Gegenwart von 21,6 g (0,156 mol) Kaliumcarbonat unter Rückfluß gekocht.
   Nach 12 h destilliert man 500 ml Methanol ab und kühlt auf Raumtemperatur. Es kristallisieren 35,5 g (68% d. Th.) des Produkts als orangerote Kristalle vom Schmp. 238,0-239,5°C aus.
   C₁₆H₂₀N₆O₃S (376,44)
   Rf = 0,62 (Laufmittel F)
c) 6-[4-[4-(Methylthio-iminomethylen)piperazin-1-yl]-3-nitro-phenyl]-4,5-dihydro-5-methyl-3(2H)-pyridazinon-hydrojodid
   Eine Suspension von 42,4 g (0,113 mol) der wie unter b) erhaltenen Verbindung in 300 ml Dimethylformamid wird tropfenweise mit 8,0 ml (0,127 mol) Methyljodid in 20 ml DMF versetzt.
   Die klare Lösung wird noch 4 h bei RT gerührt.
   Nach dem Abdestillieren des Dimethylformamids im Feinvakuum verbleibt ein rotes Öl, aus dem nach Zugabe von 200 ml Ethanol 52,7 g (90% d. Th.) eines orangegelben Feststoffs kristallisieren, der einen Schmelzbereich von 179,8-181,3°C hat.
   C₁₇H₂₃INO₃S (518,37)
   Rf = 0,58 (Laufmittel F)
d) 6-[4-[4-[3-(1H-Imidazol-4-yl)propylamino-iminomethylen]piperazin-1-yl]-3-nitro-phenyl]-4,5-dihydro-5-methyl-3(2H)-pyridazinon
   2,07 g (4 mmol) der wie unter c) erhaltenen Verbindung werden mit 0,50 g (4 mmol) 3-(1H-Imidazol-4-yl)-propylamin in 10 ml Pyridin 15 h bei RT gerührt.
   Man versetzt mit 10 ml 10%-iger wäßriger Kaliumcarbonat-Lösung und extrahiert mit insgesamt 50 ml Chloroform/Methanol (80/20 vv). Nach dem Abziehen der organischen Lösungsmittel im Vakuum versetzt man den Rückstand mit 20 ml Ethanol. Es kristalliereren 1,4 g (75% d. Th.) der Titelverbindung als gelbes Pulver vom Schmelzpunkt 225-226°C.
   C₂₂H₂₉N₉O₃ (467,54)
   RF = 0,38 (Laufmittel D)

- ¹H-NMR-Daten (DMSO-d₆, TMS als interner Standard): δ= 1,06 (d) 3H
1,83 (q) 2H
2,25 (d) 1H
2,56 (t) 2H
2,71 (dd) 1H
3,24 (m) 6H
3,42 (m) 1H
3,58 (m) 4H
6,63 (s) 1H
7,37 (d) 1H
7,59 (s) 1H
7,4-8,4 (breit) 2H,
austauschbar mit D₂O,
7,99 (dd) 1H
8,21 (d) 1H
11,05 (s) 1H
11,8 (breit) 1H,
austauschbar mit D₂O, ppm

### Beispiel 4

### N¹-[3-(1H-Imidazol-4-yl)propyl]-N³-[4-(4-methyl-6-oxo-1,4,5,6-tetrahydro-pyridazin-3-yl)-3-nitro-phenyl]-guanidin

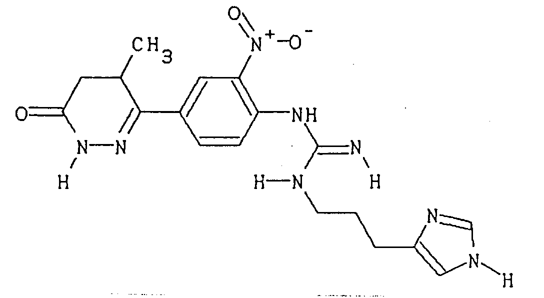

a) N¹-Benzoyl-N²-[4-(4-methyl-6-oxo-1,4,5,6-tetrahydro-pyridazin-3-yl)-3-nitro-phenyl]-thioharnstoff
   10,0 g (40,3 mmol) 6-(4-Amino-3-nitro-phenyl)-4,5-dihydro-5-methyl-3(2H)-pyridazinon werden mit 7,2 g (44,3 mmol) Benzoylisothiocyanat in 300 ml Dioxan 10 h unter Rückfluß gekocht. Das Lösungsmittel wird im Vakuum abgezogen, und der Rückstand in 200 ml Ethanol 30 min unter Rückfluß gekocht. Nach dem Abkühlen auf RT und Absaugen erhält man 14,0 g (84 % d. Th.) eines orangegelben Pulvers vom Schmp. 204,9-205,4°C.
   C₁₉H₁₇N₅O₄S (411,44)
   Rf = 0,69 (Laufmittel A)
b) 6-(4-Cyanamino-3-nitro-phenyl)-4,5-dihydro-5-methyl-3(2H)-pyridazinon
   10,0 g (24,3 mmol) der wie unter a) erhaltene Verbindung werden in 200 ml Methanol und 35 ml Wasser mit 3,4 g (24,3 mmol) Kaliumcarbonat 2 h unter Rückfluß gekocht. Die dunkelrote Lösung wird abgekühlt, man saugt vom ausgefallenen Feststoff ab und wäscht mit wenig Methanol nach. Der Filterkuchen wird in 70 ml heißem Wasser gelöst und mit Eisessig bis pH 5 angesäuert.
   Nach erneutem Absaugen in der Kälte erhält man 3,4 g (51 % d. Th.) hellgelber Kristalle, die bei 234,5-235,9°C schmelzen.
   C₁₂H₁₁N₅O₃ (273,29)
   Rf = 0,41 (Laufmittel A)
c) N¹-[3-(1H-Imidazol-4-yl)propyl]-N³-[4-(4-methyl-6-oxo-1,4,5,6-tetrahydro-pyridazin-3-yl)-3-nitro-phenyl]-guanidin
   Zu einer Lösung von 1,8 g (6,6 mmol) des wie unter b) erhaltenen Feststoffs und 1,2 g ( 9,9 mmol) 3-(1H-Imidazol-4-yl)-propylamin in 40 ml Methoxyethanol gibt man 1,0 g einer 33%-igen isopropanolischen Salzsäure.
   Man kocht 3 h unter Rückfluß, kühlt ab, versetzt mit 10 ml einer gesättigten, wäßrigen Kaliumcarbonat-Lösung und extrahiert mit Tetrahydrofuran.
   Nach dem Abziehen des Lösungsmittels unter vermindertem Druck chromatographiert man den Rückstand an Kieselgel (Laufmittel E).
   Die Hauptfraktionen werden eingeengt, der Rückstand zwischen wäßriger Kaliumcarbonat-Lösung und Tetrahydrofuran verteilt und die organische Phase eingeengt.
   Durch Behandeln des Rückstands mit Ethanol erhält man 1,1 g (42 % d. Th.) eines orangegelben Pulvers, das ab 155°C erweicht und 1/3 Molequivalent Ethanol enthält.
   C₁₈H₂₂N₈O₃ x 1/3 C₂H₆O (413,78)
   Rf = 0,65 (Laufmittel D)

- ¹H-NMR-Daten (DMSO-d₆, TMS als interner Standard): δ= 1,07 (d) 3H
1.75 (q) 2H
2,23 (d) 1H
2,56 (m) 2H
3,13 (q) 2H
3,43 (m) 1H
5,54 (s) 2H,
austauschbar mit D₂O,
6,09 (t) 1H,
austauschbar mit D₂O,
6,74 (s) 1H
7,02 (d) 1H
7,49 (s) 1H
7,80 (dd) 1H
8,06 (d) 1H
10,94 (s) 1H
11,79 (breit) 1H,
austauschbar mit D₂O,
ppm

### Beispiel 5

### 6-[4-[4-[3-(1H-Imidazol-4-yl)propylamino-iminomethylen]piperazin-1-yl]-3-nitro-phenyl]-4,5-dihydro-3(2H)-pyridazinon

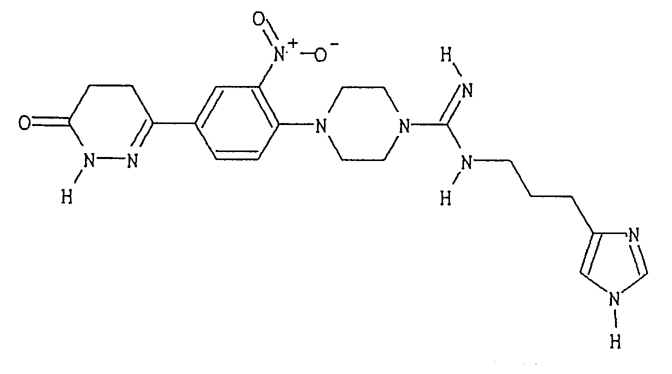

a) 6-[4-[4-(Benzoylamino-thiocarbonyl)piperazin-1-yl]-3-nitro-phenyl]-4,5-dihydro-3(2H)-pyridazinon
   Zu einer Suspension von 8,8 g (29 mmol) 6-[4-(1-Piperazinyl)-3-nitro-phenyl]-4,5-dihydro-3(2H)-pyridazinon in 300 ml Chloroform tropft man bei RT eine Lösung von 5,2 g (32 mmol) Benzoylisothiocyanat in 20 ml Chloroform.
   Man rührt 2 h bei 30°C, kühlt im Eisbad und saugt vom ausgefallenen Feststoff ab. Nach dem Trocknen verbleiben 12,5 g (93 % d. Th.) orangefarbener Kristalle vom Schmp. 140-141,5°C.
   C₂₂H₂₂N₆O₄S (466,52)
   Rf = 0,72 (Laufmittel F)
b) 6-[4-[4-(Amino-thiocarbonyl)piperazin-1-yl]-3-nitro-phenyl]-4,5-dihydro-3(2H)-pyridazinon
   12,0 g (25,7 mmol) der wie unter a) erhaltenen Benzoylverbindung werden mit 5,4 g (39 mmol) Kaliumcarbonat in 210 ml Methanol und 35 ml Wasser unter Rückfluß gekocht.
   Nach 22 h läßt man auf RT abkühlen, wobei ein Feststoff auskristallisiert.
   Nach Absaugen und Nachwaschen mit wenig Methanol erhält man 7,19 g (76 % d. Th.) eines orangenen Feststoffs vom Schmp. 222,5-223,8°C.
   C₁₅H₁₈N₆O₃S (362,41)
   Rf = 0,51 (Laufmittel F)
c) 6-[4-[4-(Methylthio-iminomethylen)piperazin-1-yl]-3-nitro-phenyl]-4,5-dihydro-3(2H)-pyridazinon-hydrojodid
   6,7 g (18,5 mmol) des wie unter b) erhaltenen Thioharnstoffs werden in 500 ml CHCl₃ und 120 ml CH₃OH suspendiert und mit 7,5 ml Methyljodid versetzt.
   Man kocht 5 h unter Rückfluß, läßt das Reaktionsgemisch abkühlen, saugt ab und wäscht mit wenig Chloroform nach.
   Man erhält 6,8 g (73 % d. Th.) gelber Kristalle vom Schmp. 209-212°C.
   C₁₆H₂₁IN₆O₃S (504,34)
   Rf = 0,65 (Laufmittel B)
d) 6-[4-[4-[3-(1H-Imidazol-4-yl)propylamino-iminomethylen] piperazin-1-yl]-3-nitro-phenyl]-4,5-dihydro-3(2H)-pyridazinon
   3,0 g (5,9 mmol) 6-[4-[4-(Methylthio-iminomethylen)piperazin-1-yl]-3-nitro-phenyl]-4,5-dihydro-3(2H)-pyridazinon werden mit 1,1 g (8,9 mmol) 3-(1H-Imidazol-4-yl)-propylamin in 100 ml Acetonitril 2 h unter Rückfluß gekocht.
   Nach dem Abkühlen wird das Reaktionsgemisch mit 40 ml einer gesättigten wäßrigen Kaliumcarbonat-Lösung versetzt, die organische Phase abgetrennt und die wäßrige mit 50 ml Chloroform/Methanol (80/2 v/v) extrahiert. Die organischen Phasen werden vereinigt, eingeengt, und der Rückstand wird an Kieselgel chromatographiert (Laufmittel F).
   Die Hauptfraktionen werden vereinigt und eingeengt. Der Rückstand wird mit ges. Kaliumcarbonat-Lösung versetzt und mit Chloroform /Methanol (80/20 v/v) extrahiert. Das Lösungsmittel wird im Vakuum eingedampft und das verbleibende Öl aus wenig Isopropanol kristallisiert. Man erhält 0,56 g (21 % d. Th.) eines orangeroten Pulvers vom Schmelzpunkt 204,8-205,4°C.
   C₂₁H₂₇N₉O₃ (453,51)
   Rf = 0,40 (Laufmittel D)

- ¹H-NMR-Daten (DMSO-d₆, TMS als interner Standard): δ= 1,73 (m) 2H
2,4 - 2,6 (m) 6H
2,9 (m) 2H
3,0 (m) 4H
3,3 (m) 4H
3,0 - 3.8 (breit) 3H,
austauschbar mit D₂O,
6,69 (s) 1H
7,34 (d) 1H
7,48 (s) 1H
7,92 (dd) 1H
8,13 (s) 1H
10,95 (breit) 1H,
austauschbar mit D₂O, ppm

### Beispiel 6

### N¹-[3-(1H-Imidazol-4-yl)propyl]-N³-[3-[4-[4-(4-methyl-6-oxo-1,4,5,6-tetrahydro-pyridazin-3-yl)-2-nitro-phenyl] piperazin-1-yl]propyl]-guanidin

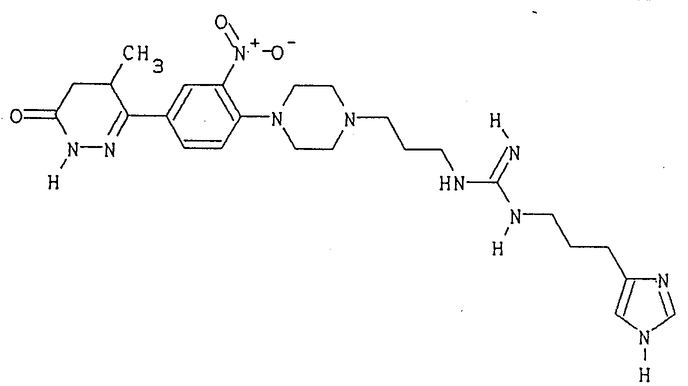

a) 6-[3-Nitro-4-[4-(3-phthalimido-propyl)piperazin-1-yl] phenyl]-5-methyl-4,5-dihydro-3(2H)-pyridazinon
   31,63 g (0,1 mol) 6-[4-(1-Piperazinyl-3-nitro-phenyl]-4,5-dihydro-5-methyl-3(2H)-pyridazinon und 26,81 g (0,1 mol) 1-(3-Brompropyl)-phthalimid werden in 500 ml DMF in Gegenwart von 13,8 g (0,1 mol) Kaliumcarbonat und 0,4 g Kaliumjodid 24 h bei 100°C gerührt.
   Man läßt auf 40°C abkühlen und gießt auf 1,6 l Eiswasser. Nach 1h saugt man vom ausgefallenen Feststoff ab, wäscht mit Wasser nach und trocknet im Vakuum.
   Die Umkristallisation aus Ethanol ergibt 26 g (52 % d.Th.) orangefarbener Kristalle vom Schmelzpunkt 179,5-180°C.
   C₂₆H₂₈N₆O₅ (504,55)
   Rf = 0,28 (Laufmittel A)
b) 6-[4-[4-(3-Aminopropyl)piperazin-1-yl]-3-nitro-phenyl]-5-methyl-4,5-dihydro-3(2H)-pyridazinon
   32,0 g (64 mmol) der wie unter a) erhaltenen Phthalimidoverbindung werden mit 30 ml Hydrazinhydrat (80 % in Wasser) in 700 ml Ethanol 5 h unter Rückfluß gekocht.
   Das Ethanol wird weitgehend im Vakuum abdestilliert, der Rückstand mit 200 ml Wasser versetzt und bis pH 2 mit konz. Salzsäure angesäuert.
   Man saugt vom Feststoff ab, stellt das Filtrat alkalisch (pH 12) und extrahiert mit Dichlormethan.
   Die organische Phase wird eingeengt und der Rückstand aus Isopropanol kristallisiert.
   Die Ausbeute beträgt 16,0 g (67 % d. Th.) eines orangeroten Pulvers vom Schmp. 110-111°C.
   C₁₈H₂₆N₆O₃ (374,45)
   Rf = 0,27 (Laufmittel C)
c) S-Methyl-N-[3-[4-[4-(4-methyl-6-oxo-1,4,5,6-tetrahydro-pyridazin-3-yl)-2-nitro-phenyl]piperazin-1-yl]propyl]-isothioharnstoff-hydrojodid
   11,23 g (30 mmol) des wie unter b) erhaltenen Amins werden mit 8,22 g (33 mmol) Dithiocarbimidsäure-S,S-dimethylester-hydrojodid in 240 ml Acetonitril 5 h unter Rückfluß gekocht. Man engt im Vakuum ein und rührt den Rückstand in 200 ml Ethylacetat. Nach dem Absaugen erhält man 17,2 g (100 % d. Th) eines roten amorphen Pulvers, das ausreichend rein ist für die weitere Umsetzung.
   C₂₀H₃₀IN₇O₃S (575,47)
   Rf = 0,4 (Laufmittel C)
d) N¹-[3-(1H-Imidazol-4-yl)propyl]-N³-[3-[4-[4-(4-methyl-6-oxo-1,4,5,6-tetrahydro-pyridazin-3-yl)-2-nitro-phenyl] piperazin-1-yl]propyl]-guanidin
   2,88 g (5 mmol) des wie unter c) erhaltenen Isothiouronium-Salzes werden mit 0,63 g (5 mmol) 3-(1H-Imidazol-4-yl)-propylamin in 10 ml DMF 12 h bei RT gerührt.
   Nach Zugabe von 5 ml einer ges. wäßrigen Kaliumcarbonatlösung extrahiert man mit Dichlormethan/Methanol (80/20 v/v). Die organische Phase wird eingeengt und der Rückstand an Kieselgel chromatographiert (Laufmittel D).
   Die Hauptfraktionen werden vereinigt und eingeengt, der Rückstand mit wäßriger Kaliumcarbonat-Lösung versetzt und mit Chloroform/Methanol (80/20 v/v) extrahiert.
   Nach Einengen der organischen Phasen erhält man 0,32 g (12 % d. Th.) eines orangeroten Schaums mit einem Erweichungsbereich von 117-119°C.
   C₂₅H₃₆N₁₀O₃ (524,63)
   Rf = 0,32 (Laufmittel D)

- ¹H-NMR-Daten (DMSO-d₆, TMS als interner Standard): δ=1,05 (d) 3H
1,70 (m) 4H
2,1 - 2,9 (m) 6H
3,08 (m) 9H
3,36 (m) 4H
6,78 (s) 1H
7,31 (d) 1H
7,53 (s) 1H
7,4 - 8,3 (breit) 1H,
austauschbar mit D₂O,
8,05 (dd) 1H
8,14 (d) 1H
9,2 (breit) 1H
und 11,8 (breit) 1H,
austauschbar mit D₂O,
11,03 (s) 1H ppm

### Beispiel 7

### 6-[4-[4-[3-(1H-Imidazol-4-yl)propylamino-carbonyl]piperazin-1-yl]-3-nitro-phenyl]-4,5-dihydro-5-methyl-3(2H)-pyridazinon

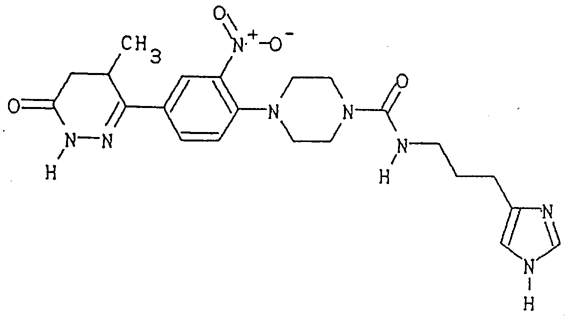

3,0 g (9,5 mmol) 6-[4-(1-Piperazinyl)-3-nitro-phenyl]-4,5-dihydro-5-methyl-3(2H)-pyridazinon werden mit 1,7 g (10,5 mmol) Carbonyl-diimidazol in 30 ml DMF 2 h bei RT gerührt.
Anschließend versetzt man mit 1,88 g (15 mmol) 3-(1H-Imidazol-4-yl)-propylamin und erhitzt 8 h auf 70°C.
Nach dem Abkühlen gibt man 10 ml einer gesättigten wäßrigen Kaliumcarbonat-Lösung zu und extrahiert mit Chloroform. Die organische Phase wird im Vakuum eingedampft und der Rückstand an Kieselgel chromatographiert (Laufmittel F).
Durch Einengen der Hauptfraktionen und Kristallisation des Rückstands erhält man 2,3 g (52 % d. Th.) orangener Kristalle, die einen Schmelzpunkt von 182°C haben.
C₂₂H₂₈N₈O₄ (468,52)
Rf = 0,36 (Laufmittel F)
- ¹H-NMR-Daten (DMSO-d₆, TMS als interner Standard): δ= 1,06 (d) 3H
1,70 (q) 2H
2,24 (d) 1H
2,45 (m) 2H
2,69 (dd) 1H
3,06 (m) 4H
3,4 (m) 7H
6,68 (t) 1H,
austauschbar mit D₂O,
6,74 (s) 1H
7,36 (d) 1H
7,50 (s) 1H
7,96 (dd) 1H
11,04 (s) 1H
11,8 (breit) 1H,
austauschbar mit D₂O, ppm

### Beispiel 8

### 6-[4-[4-[3-(1H-Imidazol-4-yl)propylamino-(ethoxycarbonyl) iminomethylen]piperazin-1-yl]-3-nitro-phenyl]-4,5-dihydro-5-methyl-3(2H)-pyridazinon

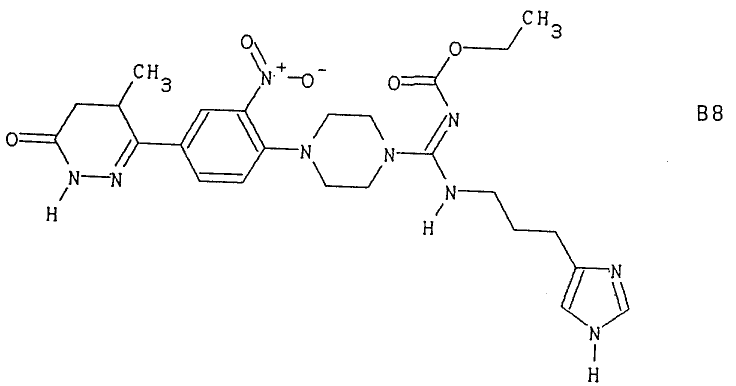

2,7 g (9,5 mmol) N-Ethoxycarbonyl-imidokohlensäurediphenylester werden in eine Suspension von 3,0 g (9,4 mmol) 6-[3-Nitro-4-(1-piperazinyl)-phenyl]-4,5-dihydro-5-methyl-3(2H)-pyridazinon in 30 ml Tetrahydrofuran eingetragen. Nach 20-stündigem Rühren bei Raumtemperatur wird die Lösung filtriert und das Filtrat i. Vak. eingedampft. Der Rückstand wird in 40 ml Acetonitril aufgenommen. Nach Zugabe von 1,2 g (9,6 mmol) 3-(1H-Imidazol-4-yl)-propylamin wird das Gemisch 3,5 Stunden unter Rückfluß gekocht. Die Lösung wird i. Vak. eingedampft und der erhaltene Rückstand an Kieselgel mit Laufmittel G chromatographiert.
Die orangefarbene polare Fraktion wird i. Vak. eingedampft, der Rückstand in 10 ml gesättigter Kaliumcarbonat-Lösung aufgenommen und die wässrige Phase mit 3 x 10 ml Isopropanol extrahiert. Die vereinigten organischen Phasen ergeben nach Trocknen mit Kaliumcarbonat, Filtrieren und Eindampfen i. Vak. 3,83 g (75%) eines orangegelben, amorphen Feststoffs.
C₂₅H₃₃N₉O₅ (539,60)
Rf = 0,34 (Laufmittel D)
- ¹H-NMR-Daten (DMSO-d₆, TMS als interner Standard): δ= 1,04 (d) 3H
1,14 (t) 3H
1,78 (m) 2H
2,20-2,72 (m) 2H
2,49 (t) 2H
3,0 -3,6 (m) 10H
3,78 (quin) 1H
3,91 (q) 2H
6,74 (s) 1H
7,34 (d) 1H
7,52 (s) 1H
7,96 (dd) 1H
8,19 (d) 1H ppm

### Beispiel 9

### N¹-[3-(1H-Imidazol-4-yl)propyl]-N³-[3-[4-(4-methyl-6-oxo-1,4,5,6-tetrahydropyridazin-3-yl)-2-nitro-anilino]propyl]-guanidin

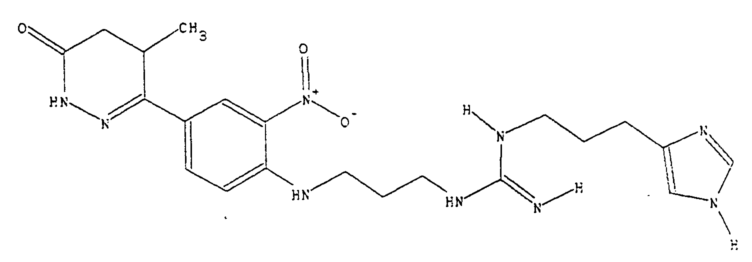

a) N¹-Benzoyl-N²-[3-[4-(4-methyl-6-oxo-1,4,5,6-tetrahydropyridazin-3-yl)-2-nitro-anilino]propyl]-thioharnstoff
   Aus 7,00 g (22,9 mmol) N-[4-(4-Methyl-6-oxo-1,4,5,6-tetrahydropyridazin-3-yl)-2-nitro-phenyl]-1,3-propandiamin und 3,75 g (23 mmol) Benzoylisothiocyanat werden analog zu Beispiel 1a) 7,51 g (70 %) eines orangegelben Feststoffs vom Schmp. 118-120°C erhalten.
   C₂₂H₂₄N₆O₄ (468,54)
   Rf = 0,93 (Laufmittel F)
b) N-[3-[4-(4-Methyl-6-oxo-1,4,5,6-tetrahydropyridazin-3-yl)-2-nitro-anilino]-propyl]-thioharnstoff
   Aus 7,5 g (16,0 mmol) des wie unter a) hergestellten Benzoylthioharnstoffs werden analog zu Beispiel 1b) 4,0 g (69 %) eines orangegelben Feststoffs vom Schmelzpunkt 199-203°C erhalten.
   C₁₅H₂₀N₆O₃S (364,43)
   Rf = 0,53 (Laufmittel F)
c) N-[3-[4-(4-Methyl-6-oxo-1,4,5,6-tetrahydropyridazin-3-yl)-2-nitro-anilino]propyl]-S-methyl-isothiuroniumjodid
   2,00 g (5,5 mmol) des Thioharnstoffs aus Stufe b) werden analog zu Beispiel 1c) mit 0,40 ml (6,4 mmol) Methyljodid methyliert. Nach Umkristallisieren des Rohprodukts aus Ethylacetat/Methanol (7:3) verbleiben 0,70 g (25 %) eines orangegelben Feststoffs vom Schmp. 152-154°C.
   C₁₆H₂₃JN₆O₃S (506,37)
   Rf = 0,34 (Laufmittel G)
d) N¹-[3-(1H-Imidazol-4-yl)propyl]-N³-[3-[4-(4-methyl-6-oxo-1,4, 5,6-tetrahydropyridazin-3-yl)-2-nitro-anilino]propyl]-guanidin
   0,60 g (1,18 mmol) des wie unter c) erhaltenen Isothiuroniumjodids werden mit 0,15 g (1,19 mmol) 3-(1H-Imidazol-4-yl)-propylamin in 20 ml Dioxan 4 Stunden unter Rückfluß gekocht. Der nach Eindampfen der Lösung im Vakuum erhaltene Rückstand wird an Kieselgel mit Laufmittel D chromatographiert. Die Produktfraktionen werden vereinigt, i. Vak. eingedampft und der Rückstand in 10 ml gesättigter Kaliumcarbonat-Lösung aufgenommen. Nach Extraktion mit 3 x 10 ml Isopropanol, Trocknen der vereinigten organischen Phasen und Eindampfen i. Vak. verbleiben 0,16 g (30 %) eines orangegelben, amorphen Feststoffs.
   C₂₁H₂₉N₉O₃ (455,52)
   Rf = 0,42 (Laufmittel D)

- ¹H-NMR-Daten (DMSO-d₆, TMS als interner Standard): δ= 1,05 (d) 3H
1,6-1,95 (m) 4H
2,1-3,6 (m) 11H
6,75 (s) 1H
7,18 (d) 1H
7,53 (s) 1H
7,95 (dd) 1H
8,38 (d) 1H
8,0 und 8,5 (breit) 6H,
austauschbar mit D₂O, ppm.

### Beispiel 10

### N¹-[3-(1H-Imidazol-4-yl)propyl]-N³-[1-[4-(4-methyl-6-oxo-1,4,5,6-tetrahydropyridazin-3-yl)-2-nitro-phenyl]-piperidin-4-yl]-guanidin

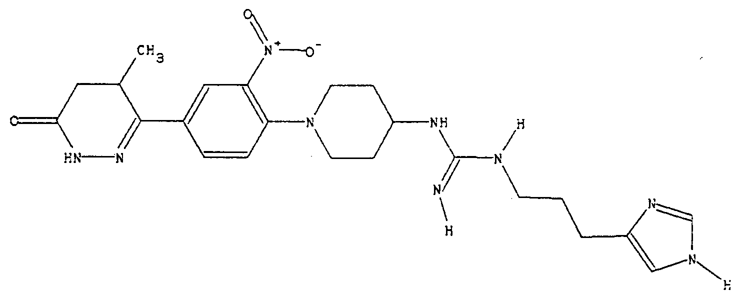

a) N¹-Benzoyl-N²-[1-[4-(4-methyl-6-oxo-1,4,5,6-tetrahydropyridazin-3-yl)-2-nitro-phenyl]piperidin-4-yl]-thioharnstoff
   Zu einer Suspension von 1,20 g (3,62 mmol) 6-[4-(4-Aminopiperidin-1-yl)-3-nitro-phenyl]-4,5-dihydro-5-methyl-3(2H)-pyridazinon in 30 ml Acetonitril werden 0,60 g (3,67 mmol) Benzoylisothiocyanat in 5 ml Acetonitril getropft. Nach zweistündigem Rühren bei Raumtemperatur wird der ausgefallene Feststoff abgesaugt, mit 10 ml Acetonitril gewaschen und i. Vak. getrocknet. Es werden 1,09 g (61 %) eines gelben Feststoffs vom Schmp. 192-194°C erhalten.
   C₂₄H₂₆N₆O₄S (494,58)
   Rf = 0,68 (Laufmittel H)
b) N-[1-[4-(4-Methyl-6-oxo-1,4,5,6-tetrahydropyridazin-3-yl)-2-nitro-phenyl]-piperidin-4-yl]-thioharnstoff
   Aus 1,00 g (2,02 mmol) des wie unter a) hergestellten Benzoylthioharnstoffs erhält man durch Hydrolyse analog zu Beispiel 1b) 0,73 g (93 %) eines gelben Feststoffs vom Schmp. 222-224°C.
   C₁₇H₂₂N₆O₃S (390,47)
   Rf = 0,58 (Laufmittel F)
c) N-[1-[4-(4-Methyl-6-oxo-1,4,5,6-tetrahydropyridazin-3-yl)-2-nitro-phenyl]-piperidin-4-yl]-S-methyl-isothiuroniumjodid
   0,60 g (1,53 mmol) des Thioharnstoffs aus Stufe b) werden mit 0,11 ml (1,76 mmol) Methyljodid in 30 ml Methanol 20 Std. bei Raumtemperatur gerührt. Der nach Abdampfen des Lösungsmittels i. Vak. erhaltene Schaum (0,85 g) ist DC-rein und wird ohne weitere Aufarbeitung in die nächste Stufe eingesetzt.
   C₁₈H₂₅JN₆O₃S (532,41)
   Rf = 0,51 (Laufmittel F)
d) N¹-[3-(1H-Imidazol-4-yl)propyl]-N³-[1-[4-(4-methyl-6-oxo-1,4,5,6-tetrahydropyridazin-3-yl)-2-nitro-phenyl]piperidin-4-yl]-guanidin
   0,75 g (1,41 mmol) N-[1-[4-(4-Methyl-6-oxo-1,4,5,6-tetrahydropyridazin-3-yl)-2-nitro-phenyl]piperidin-4-yl]-S-methyl-isothiuroniumjodid und 0,20 g (1,59 mmol) 3-(1H-Imidazol-4-yl) propylamin werden zusammen in 30 ml Acetonitril 3 Stunden unter Rückfluß gekocht. Der nach Abdampfen des Lösungsmittels i. Vak. erhaltene Rückstand wird wie in Beispiel 9d) chromatographisch gereinigt. Man erhält 0,57 g (84 %) eines orangegelben, amorphen Feststoffs.
   C₂₃H₃₁N₉O₃ (481,56)
   Rf = 0,57 (Laufmittel D)

- ¹H-NMR-Daten (DMSO-d₆, TMS als interner Standard): δ= 1,05 (d) 3H
1,4- 3,7 (m) 18H
6,77 (s) 1H
7,34 (d) 1H
7,54 (s) 1H
7,93 (dd) 1H
8,15 (d) 1H
8,4 (breit) 5H,
austauschbar mit D₂O ppm.

### Beispiel 11

### 6-[4-[4-[3-(1H-Imidazol-4-yl)-propylamino-iminomethylen]-piperazin-1-yl]-3-nitro-phenyl]-5-hydroxymethyl-4,5-dihydro-3(2H)-pyridazinon

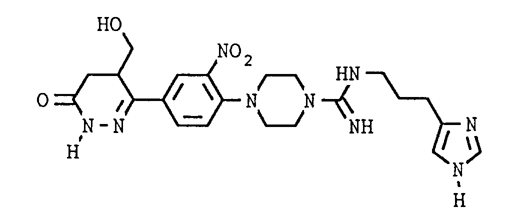

Die Titelverbindung wird analog zu Beispiel 3d) aus 1,60 g (3 mmol) 6-[4-[4-(Methylthio-iminomethylen)-piperazin-1-yl]-3-nitro-phenyl]-4,5-dihydro-5-hydroxymethyl-3(2H)-pyridazinon-hydrojodid und 0,40 g (3,4 mmol) 3-(1H-Imidazol-4-yl)-propylamin in 8 ml Dimethylformamid erhalten.
Das Rohprodukt wird an Kieselgel mit Laufmittel E chromatographisch gereinigt und aus Ethanol umkristallisiert.
Man erhält 0,22 g (15 %) orangegelbe Kristalle vom Schmp. 196,7-197,3 °C.
C₂₂H₂₉N₉O₄ (483,53)
Rf = 0,22 (Laufmittel D)
- ¹H-NMR-Daten (DMSO-d₆, TMS als interner Standard): δ= 1,73 (quin) 2H
2,43-2,75(m) 4H
2,96 (t) 4H
3,0-3,4 (m) 12H,
1H austauschb. mitD₂O
6,69 (s) 1H
7,33 (d) 1H
7,48 (s) 1H
7,95 (dd) 1H
8,17 (d) 1H
ppm.

### Beispiel 12

### 6-[3-Fluor-4-[4-[3-(1H-imidazol-4-yl)-propylamino-iminomethylen]-piperazin-1-yl]-phenyl]-4,5-dihydro-5-methyl-3(2H)-pyridazinon

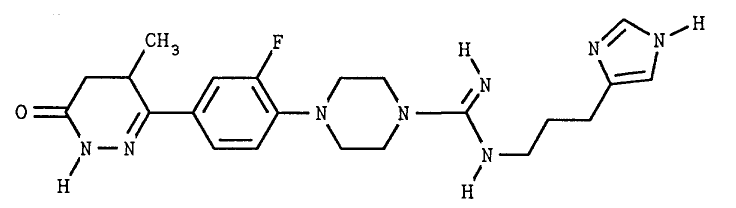

Analog zu Beispiel 3d) werden aus 0,90 g (1,83 mmol) 6-[3-Fluor-4-[4-(methylthio-iminomethylen)-piperazin-1-yl]-phenyl]-4,5-dihydro-5-methyl-3(2H)-pyridazinon-hydrojodid und 0,23 g (1,83 mmol) 3-(1H-Imidazol-4-yl)-propylamin nach chromatographischer Reinigung des Rohprodukts an Kieselgel mit Laufmittel D 0,54 g (67 %) eines blaßgelblichen, amorphen Feststoffes erhalten.
C₂₂H₂₉FN₈O (440,53)
Rf = 0,50 (Laufmittel D)
- ¹H-NMR-Daten (DMSO-d₆, TMS als interner Standard): δ= 1,11 (d) 3H
1,78 (quin) 2H
2,33-2,67(m) 4H
2,9-3,5 (m) 11H
6,75 (s) 1H
7,06 (t) 1H
7,40-7,58(m) 4H,
1H austauschb. mit D₂O
7,9 (breit) 2H,
austauschbar mit D₂O
10,8 (breit) 1H,
austauschbar mit D₂O ppm.

### Beispiel 13

### 6-[3-Cyano-4-[4-[3-(1H-imidazol-4-yl)-propylamino-iminomethylen]-piperazin-1-yl]-phenyl]-4,5-dihydro-3(2H)-pyridazinon

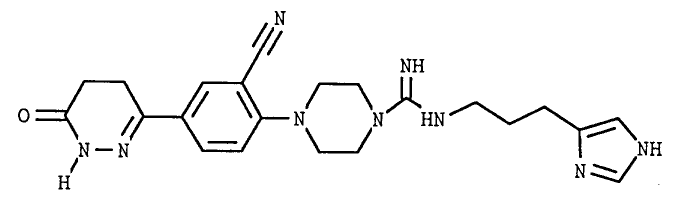

1,00 g (2,1 mmol) 6-[3-Cyano-4-[4-(methylthio-iminomethylen)-piperazin-1-yl]-phenyl]-4,5-dihydro-3(2H)-pyridazinon-hydrojodid und 0,36 g (2,88 mmol) 3-(1H-Imidazol-4-yl)-propylamin werden in 20 ml Acetonitril und 10 ml Dimethylformamid 11 Stunden unter Rückfluß gekocht.
Nach Abdampfen des Lösungsmittels i. Vak. wird der erhaltene Rückstand an Kieselgel mit Laufmittel G chromatographiert. Der aus der Hauptfraktion nach Eindampfen erhaltene Rückstand wird in 10 ml gesättigter Kaliumcarbonatlösung aufgenommen.
Dabei kristallisieren 0,52 g (57 %) eines farblosen Feststoffes vom Schmp. 263,8 - 265,6 °C (Zers.).
C₂₂H₂₇N₉O (433,52)
Rf = 0,25 (Laufmittel E)
- ¹H-NMR-Daten (DMSO-d₆, TMS als interner Standard): δ= 1,85 (m) 2H
2,31-2,63(m) 4H
2,95 (t) 2H
3,0-3,8 (m) 10H
6,83 (s) 1H
7,23 (d) 1H
7,59 (s) 1H
7,95-8,04(s+d) 2H
8,7 (breit) 2H,
austauschbar mit D₂O
11,0 (s) 1H,
austauschbar mit D₂O ppm.

### Beispiel 14

### 6-[3-Cyano-4-[4-[3-(1H-imidazol-4-yl)-propylamino-iminomethylen]-piperazin-1-yl]-phenyl]-4,5-dihydro-5-methyl-3(2H)-pyridazinon

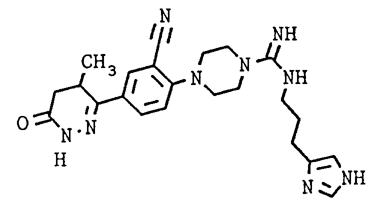

Zu einer Lösung von 1,80 g (5 mmol) 6-[3-Cyano-4-[4-(aminothiocarbonyl)-piperazin-1-yl]-phenyl]-4,5-dihydro-5-methyl-3(2H)-pyridazinon in 30 ml Dimethylformamid werden 0,47 ml (7,5 mmol) Methyljodid gegeben und das Reaktionsgemisch bei Raumtemperatur gerührt.
Nach 16 Stunden werden 0,69 g (5,5 mmol) 3-(1H-imidazol-4-yl)-propylamin zugegeben und die Lösung 2 Tage weitergerührt. Man versetzt mit 20 ml 10%-iger wäßriger Kaliumcarbonatlösung und extrahiert mit 3 x 20 ml Chloroform/ Methanol (80/20 v/v.).
Nach Trocknen und Eindampfen der organischen Phasen i. Vak. wird der Rückstand an Kieselgel mit Laufmittel E chromatographiert.
Die eingedampfte Hauptfraktion wird mit Ethylacetat kristallisiert.
Es werden 1,12 g (50 %) eines farblosen Feststoffes erhalten, der nach NMR-Spektrum ca. 1/3 Mol Ethylacetat enthält und unscharf ab 140 °C schmilzt.
C₂₃H₂₉N₉O (447,55)
Rf = 0,37 (Laufmittel D)
- ¹H-NMR-Daten (DMSO-d₆, TMS als interner Standard): δ= 1,05 (d) 3H
1,80 (quin) 2H
2,11-2,78(m) 4H
3,0-3,7 (m) 11H
6,73 (s) 1H
7,21 (d) 1H
7,50 (s) 1H
7,96-8,06(m) 2H
9,1 (breit) 2H,
austauschbar mit D₂O
11,1 (breit) 1H,
austauschbar mit D₂O ppm.

### Beispiel 15

### N¹-[3-(1H-Imidazol-4-yl)propyl]-N³-[1-[4-(4-hydroxymethyl-6-oxo-1,4,5,6-tetrahydropyridazin-3-yl)-2-nitro-phenyl]-piperidin-4-yl]-guanidin

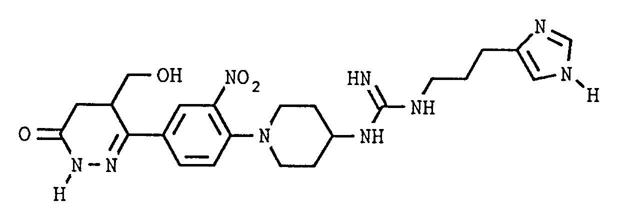

2,11 g (4,1 mmol) N-[1-[4-(4-Hydroxymethyl-6-oxo-1,4,5,6-tetrahydropyridazin-3-yl)-2-nitro-phenyl]-piperidin-4-yl]-S-methyl-isothiuroniumjodid und 0,57 g (4,5 mmol) 3-(1H-Imidazol-4-yl)-propylamin werden in 50 ml Dimethylformamid 9 Stunden bei 90 °C gerührt.
Das weitgehend i. Vak. eingeengte Reaktionsgemisch wird in 70 ml Dichlormethan/ Methanol (80/20 v/v) aufgenommen und mit 20 ml 50%-iger Kaliumcarbonatlösung ausgeschüttelt.
Die organische Phase wird mit Natriumsulfat getrocknet, filtriert und i. Vak. eingedampft. Nach chromatographischer Reinigung an Kieselgel mit Laufmittel E wird der erhaltene Feststoff aus Acetonitril kristallisiert. Man erhält 0,52 g (25 %) eines orangen Pulvers vom Schmp. 135 - 137 °C.
C₂₃H₃₁N₉O₄ (497,56)
Rf = 0,28 (Laufmittel E)
- ¹H-NMR-Daten (DMSO-d₆, TMS als interner Standard): δ= 1,45-2,0 (m) 6H
2,2-2,8 (m) 4H
2,93 (t) 2H
3,05-3,72(m) 9H,
1H austauschb. mit D₂O
6,78 (s) 1H
7,32 (d) 1H
7,54 (s) 1H
7,8 (breit) 3H,
austauschbar mit D₂O
7,94 (dd) 1H
8,16 (d) 1H
ppm.

### Beispiel 16

### N¹-[3-(1H-imidazol-4-yl)propyl]-N³-[[1-[4-(4-methyl-6-oxo-1,4,5,6-tetrahydropyridazin-3-yl)-2-nitro-phenyl]-piperidin-4-yl]methyl]-guanidin

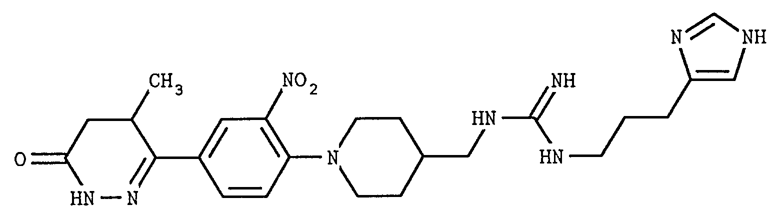

1,20 g (2,2 mmol) S-Methyl-N-[[1-[4-(4-methyl-6-oxo-1,4,5,6-tetrahydropyridazin-3-yl)-2-nitro-phenyl]-piperidin-4-yl]methyl]-isothiuroniumjodid und 0,30 g (2,4 mmol) 3-(1H-Imidazol-4-yl)-propylamin werden in 30 ml Pyridin 3 Stunden unter Rückfluß gekocht.
Der nach Abdampfen des Lösungsmittels i. Vak. erhaltene Rückstand wird wie in Beispiel 9d) chromatographisch gereinigt.
Man erhält 0,57 g (53 %) eines orangegelben Feststoffes.
C₂₄H₃₃N₉O₃ (495,59)
Rf = 0,57 (Laufmittel D)
- ¹H-NMR-Daten (DMSO-d₆, TMS als interner Standard): δ= 1,04 (d) 3H
1,2-3,5 (m) 20H
6,76 (s) 1H
7,31 (d) 1H
7,53 (s) 1H
7,92 (dd) 1H
8,12 (d) 1H
8,4 (breit) 5H,
austauschbar mit D₂O ppm.

### Beispiel 17

### N¹-[3-(1H-Imidazol-4-yl)propyl]-N³-[1-[4-(6-oxo-1,4,5,6-tetrahydropyridazin-3-yl)-2-cyano-phenyl]-piperidin-4-yl]-guanidin

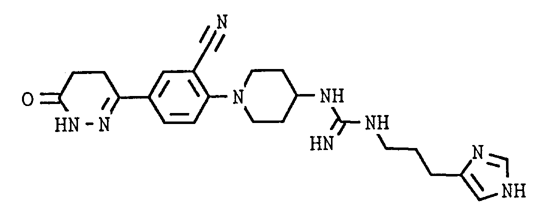

Aus 1,00 g (2,0 mmol) N-[1-[2-Cyano-4-(6-oxo-1,4,5,6-tetrahydropyridazin-3-yl)-phenyl]-piperidin-4-yl]-S-methyl-isothiuroniumjodid und 0,25 g (2,0 mmol) 3-(1H-Imidazol-4-yl)-propylamin werden analog zu Beispiel 13) nach chromatographischer Reinigung an Kieselgel mit Laufmittel G und Kristallisation mit Isopropanol/Ethylacetat 0,36 g (40 %) eines hellbeigen Feststoffes vom Schmp. 144 - 148 °C erhalten.
C₂₃H₂₉N₉O (447,55)
Rf = 0,33 (Laufmittel E)
- ¹H-NMR-Daten (DMSO-d₆, TMS als interner Standard): δ= 1,4-2,1 (m) 6H
2,28-2,6 (m) 4H
2,8-3,8 (m) 9H
6,77 (s) 1H
7,20 (d) 1H
7,53 (s) 1H
7,85-7,98(m) 2H
8,6 (breit) 4H,
austauschbar mit D₂O ppm.

### Beispiel 18

### N¹-[3-(1H-Imidazol-4-yl)propyl]-N³-[4-[4-(4-methyl-6-oxo-1,4,5,6-tetrahydropyridazin-3-yl)-2-nitro-anilino]butyl]-guanidin

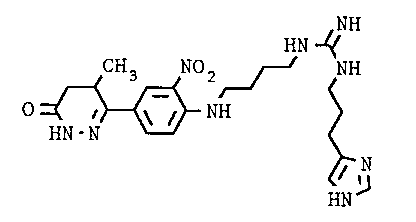

Aus 1,40 g (2,7 mmol) N-[4-[4-(4-Methyl-6-oxo-1,4,5,6-tetrahydropyridazin-3-yl)-2-nitro-anilino]butyl]-S-methyl-isothiuroniumjodid und 0,34 g (2,7 mmol) 3-(1H-Imidazol-4-yl)-propylamin werden analog zu Beispiel 1d) 0,50 g (39 %) der Titelverbindung in Form eines orangefarbenen, amorphen Feststoffes erhalten.
C₂₂H₃₁N₉O₃ (469,55)
Rf = 0,48 (Laufmittel D)
- ¹H-NMR-Daten (DMSO-d₆, TMS als interner Standard): δ= 1,06 (d) 3H
1,4-1,9 (m) 6H
2,15-2,80(m) 4H
2,95-3,53(m) 7H
6,76 (s) 1H
7,14 (d) 1H
7,54 (s) 1H
7,95 (dd) 1H
8,37 (d) 1H
8,5 (breit) 5H,
austauschbar mit D₂O ppm.

### Beispiel 19

### N¹-[3-(1H-Imidazol-4-yl)propyl]-N³-[3-[4-(4-methyl-6-oxo-1,4,5,6-tetrahydropyridazin-3-yl)-2-nitro-phenyl]propyl]-guanidin

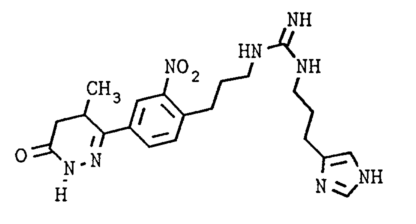

2,0 g (4 mmol) N-[3-[4-(4-Methyl-6-oxo-1,4,5,6-tetrahydropyridazin-3-yl)-2-nitro-phenyl]propyl]-S-methyl-isothuroniumjodid und 0,5 g (4 mmol) 3-(1H-Imidazol-4-yl)-propylamin werden in 15 ml Dimethylformamid 2 Tage bei Raumtemperatur gerührt.
Nach Abdampfen des Lösungsmittels im Feinvakuum wird der Rückstand mit 10 ml gesättigter Kaliumcarbonat-Lösung versetzt und mit 3x20 ml Chloroform/ Methanol (80/20 v/v) extrahiert.
Der nach Abdampfen der Lösungsmittel i. Vak. erhaltene Rückstand wird zur Reinigung an Kieseigel mit Laufmittel D chromatographiert.
Nach Aufarbeitung analog zu Beispiel 1d) werden 0,46 g (26 %) eines gelblich-beigen Pulvers vom Schmelzbereich 96-99 °C erhalten, das nach ¹H-NMR 1/3 Mol Kristallethanol enthält.
C₂₁H₂₈N₈O₃ x 1/3 C₂H₆O (440,51)
Rf = 0,36 (Laufmittel D)
- ¹H-NMR-Daten (DMSO-d₆, TMS als interner Standard): δ= 1,03-1,23(m) 3H
+ 3H (EtOH)
1,60-1,90(m) 4H
2,4-2,75 (m) 4H
2,87 (t) 2H
3,03-3,3 (m) 5H
3,38 (q) 2H (EtOH)
6,76 (s) 1H
7,52 (s) 1H
7,59 (d) 1H
8,00 (dd) 1H
8,23 (d) 1H
8,6 (breit) 4H,
austauschbar mit D₂0 ppm.

### Beispiel 20

### N¹-[3-(1H-Imidazol-4-yl)propyl]-N³-[3-[4-(4-hydroxymethyl-6-oxo-1,4,5,6-tetrahydropyridazin-3-yl)-2-nitro-anilino]propyl]-guanidin

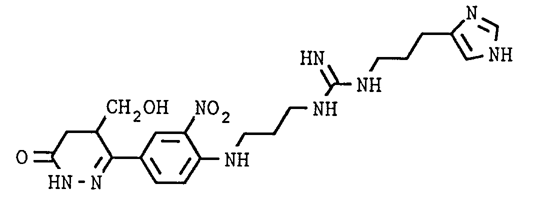

Zu einer Lösung von 1,93 g (6 mmol) N-[4-(4-Hydroxymethyl-6-oxo-1,4,5,6-tetrahydropyridazin-3-yl)-2-nitro-phenyl]-1,3-propandiamin in 10 ml Dimethylformamid werden 1,50 g (6 mmol) Dithiocarbimidsäure-S,S-dimethylester-hydrojodid gegeben.
Nach zweitägigem Rühren bei Raumtemperatur werden 1,12 g (9 mmol) 3-(1H-Imidazol-4-yl)-propylamin zugegeben und das Reaktionsgemisch 12 Stunden weitergerührt.
Nach Aufarbeitung analog Beispiel 19 (Laufmittel G) erhält man 1,10 g (39 %) eines orangefarbenen, amorphen Pulvers mit einem Schmelzbereich von 113-116 °C.
C₂₁H₂₉N₉O₄ (471,52)
Rf = 0,24 (Laufmittel G)
- ¹H-NMR-Daten (DMSO-d₆, TMS als interner Standard): δ= 1,6-2,0 (m) 4H
2,4-2,7 (m) 4H
3,1-3,6 (m) 10H
1H austauschb. mit D₂O
6,79 (s) 1H
7,21 (d) 1H
7,55 (s) 1H
7,98 (dd) 1H
8,43 (d) 1H
8,5 (breit) 5H,
austauschbar mit D₂O ppm.

### Beispiel 21

### N¹-[3-(1H-Imidazol-4-yl)propyl]-N²-[2-[4-(4-methyl-6-oxo-1,4,5,6-tetrahydropyridazin-3-yl)-2-nitro-anilino]ethyl]-harnstoff

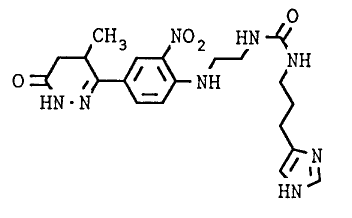

Eine Suspension von 1,98 g (6,8 mmol) N-[4-(4-Methyl-6-oxo-1,4, 5,6-tetrahydropyridazin-3-yl)-2-nitro-phenyl]-ethylendiamin in 40 ml Dioxan wird mit 1,11 g (6,84 mmol) Carbonyldiimidazol versetzt und 5 Stunden bei Raumtemperatur gerührt.
0,85 g (6,8 mmol) 3-(1H-Imidazol-4-yl)-propylamin werden zugegeben und danach wird die Suspension 3 Stunden unter Rückfluß gekocht.
Zum abgekühlten Reaktionsgemisch werden 30 ml Wasser gegeben, der ausgefallene Feststoff wird abgesaugt und das Filtrat mit 3x30 ml Dichlormethan/ Methanol (90/10 v/v) extrahiert.
Der nach Eindampfen der vereinigten organischen Phasen i. Vak. erhaltene Feststoff wird zweimal aus Methanol umkristallisiert.
Man erhält 0,35 g (12 %) orangegelbe Kristalle vom Schmp. 177-178 °C.
C₂₀H₂₆N₈O₄ (442,48)
Rf = 0,64 (Laufmittel F)
- ¹H-NMR-Daten (DMSO-d₆, TMS als interner Standard): δ= 1,06 (d) 3H
1,65 (quin) 2H
2,16-2,82(m) 4H
2,9-3,6 (m) 7H
6,08 (t) 1H,
austauschbar mit D₂O
6,17 (breit) 1H,
austauschbar mit D₂O
6,73 (s) 1H
7,22 (d) 1H
7,51 (s) 1H
7,95 (dd) 1H
8,39 (d) 1H
8,51 (t) 1H,
austauschbar mit D₂O
10,96 (s) 1H,
austauschbar mit D₂O
11,7 (breit) 1H,
austauschbar mit D₂O ppm.

### Beispiel 22

### N¹-[3-(1H-Imidazol-4-yl)propyl]-N²-[3-[4-(4-methyl-6-oxo-1,4,5,6-tetrahydropyridazin-3-yl)-2-nitro-anilino]propyl]-harnstoff

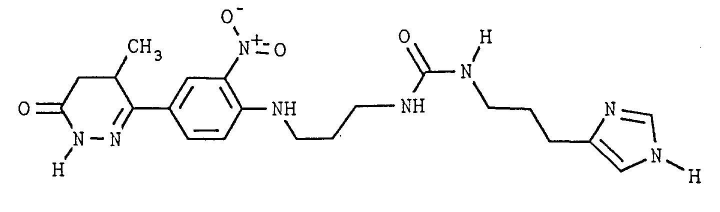

Analog zu Beispiel 21 werden aus 2,0 g (6,5 mmol) N-[4-(4-Methyl-6-oxo-1,4,5,6-tetrahydropyridazin-3-yl)-2-nitro-phenyl]-1,3-propandiamin, 1,06 g (6,5 mmol) Carbonyldiimidazol und 0,82 g (6,5 mmol) 3-(1H-Imidazol-4-yl)-propylamin nach chromatographischer Reinigung des Rohprodukts an Kieselgel mit Laufmittel Dichlormethan/ Methanol/ konz. Ammoniak (90:9:1) und Umkristallisation aus Methanol 0,51 g (17 %) eines orangegelben Feststoffes vom Schmp. 177-179 °C erhalten.
C₂₁H₂₈N₈O₄ (456,51)
Rf = 0,30 (Dichlormethan:Methanol:konz. Ammoniak 90:9:1)
- ¹H-NMR-Daten (DMSO-d₆, TMS als interner Standard): δ = 1,07 (d) 3H
1,56-1,82(m) 4H
2,17-2,76(m) 4H
2,9-3,5 (m) 7H
5,95 (t) 1H,
austauschbar mit D₂O
6,01 (t) 1H,
austauschbar mit D₂O
6,73 (s) 1H
7,13 (d) 1H
7,50 (s) 1H
7,96 (dd) 1H
8,39 (d) 1H
9,47 (t) 1H,
austauschbar mit D₂O
10,94 (s) 1H,
austauschbar mit D₂O
11,8 (breit) 1H,
austauschbar mit D₂O ppm.

### Beispiel 23

### N²-Benzoyl-N¹-[3-(1H-imidazol-4-yl)propyl]-N³-[1-[4-(4-methyl-6-oxo-1,4,5,6-tetrahydropyridazin-3-yl)-2-nitro-phenyl]-piperidin-4-yl]-guanidin

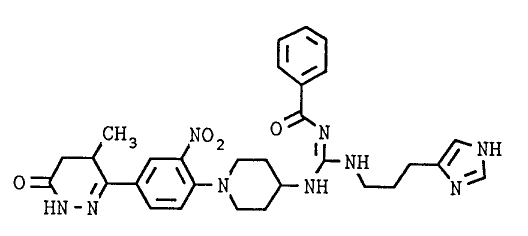

1,70 g (5,13 mmol) 6-[4-(4-Amino-piperidin-1-yl)-3-nitro-phenyl]-4,5-dihydro-5-methyl-3(2H)-pyridazinon, 1,60 g (5,04 mmol) N-Benzoyl-imidokohlensäure-diphenylester und 0,65 g (5,2 mmol) 3-(1H-Imidazol-4-yl)-propylamin werden analog zu Beispiel 8 umgesetzt.
Das Rohprodukt wird in 15 ml Methoxyethanol in der Siedehitze gelöst und nach Abkühlen der Lösung auf Raumtemperatur durch Zugabe von 20 ml Methanol ausgefällt.
Man erhält 0,58 g (20 %) orangegelbe Kristalle vom Schmp. 192-193 °C.
C₃₀H₃₅N₉O₄ (585,67)
Rf = 0,43 (Dichlormethan:Methanol:konz. Ammoniak 90:10:1)
- ¹H-NMR-Daten (CD₃OD, TMS als interner Standard): δ= 1,17 (d) 3H
1,67-2,10(m) 6H
2,32-2,86(m) 4H
3,06 (t) 2H
3,1-3,7 (m) 6H
4,9 (breit) 4H,
austauschbar mit D₂O
6,83 (s) 1H
7,28-7,53(m) 4H
7,57 (s) 1H
7,96 (dd) 1H
8,02-8,17(m) 2H
8,20 (d) 1H ppm.

### Beispiel 24

### N²-Ethoxycarbonyl-N¹-[3-(1H-imidazol-4-yl)propyl]-N³-[1-[4-(4-methyl-6-oxo-1,4,5,6-tetrahydropyridazin-3-yl)-2-nitro-phenyl]-piperidin-4-yl]-guanidin

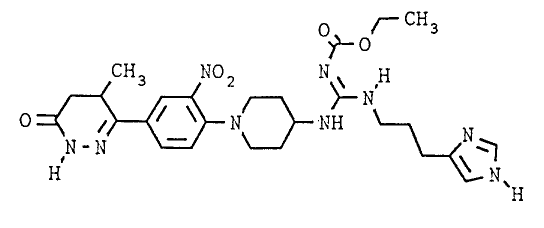

a) N¹-Ethoxycarbonyl-N²-[1-[4-(4-methyl-6-oxo-1,4,5,6-tetrahydropyridazin-3-yl)-2-nitro-phenyl]-piperidin-4-yl]-S-methyl-isothioharnstoff
   Eine Suspension von 0,90 g (1,69 mmol) N-[1-[4-(4-Methyl-6-oxo-1,4,5,6-tetrahydropyridazin-3-yl)-2-nitro-phenyl]-piperidin-4-yl]-S-methyl-isothiuroniumjodid in 20 ml Dichlormethan wird langsam mit 1,18 ml (8,46 mmol) Triethylamin versetzt und 10 Minuten gerührt.
   Zur entstandenen Lösung werden 0,20 ml (2,09 mmol) Chlorameisensäureethylester in 10 ml Dichlormethan getropft. Die Lösung wird 2 Stunden bei Raumtemperatur gerührt und dann zweimal mit 10 ml Wasser gewaschen.
   Nach Trocknen der organ. Phase und Eindampfen i. Vak. erhält man einen orangegelben Feststoff, der nach Umkristallisieren aus 15 ml Ethylacetat bei 193-195 °C schmilzt.
   Ausbeute 0,70 g (87 %)
   C₂₁H₂₈N₆O₅S (476,56)
   Rf = 0,64 (Laufmittel H)
b) N²-Ethoxycarbonyl-N¹-[3-(1H-imidazol-4-yl)propyl]-N³-[1-[4-(4-methyl-6-oxo-1,4,5,6-tetrahydropyridazin-3-yl)-2-nitro-phenyl]-piperidin-4-yl]-guanidin
   0,65 g (1,36 mmol) der unter a) erhaltenen Verbindung und 0,38 g (3,04 mmol) 3-(1H-Imidazol-4-yl)-propylamin werden in 20 ml Acetonitril 8 Stunden unter Rückfluß gekocht.
   Der nach Abdampfen des Lösungsmittels i.Vak. erhaltene Rückstand wird an Kieselgel mit Dichlormethan:Methanol:konz.
   Ammoniak (90:10:1) als Laufmittel chromatographiert.
   Die polare, orangefarbene Hauptfraktion ergibt nach Eindampfen i. Vak. 0,19 g (26 %) einen orangegelben, amorphen Feststoff.
   C₂₆H₃₅N₉O₅ (553,63)
   Rf = 0,36 (Dichlormethan:Methanol:konz. Ammoniak 90:10:1)

- ¹H-NMR-Daten (CDCl₃, TMS als interner Standard): δ= 1,24 (d) 3H
1,31 (t) 3H
1,7-2,2 (m) 6H
2,40-2,81(m) 4H
2,94-3,10(m) 2H
3,2-3,5 (m) 6H
4,12 (q) 2H
4,2 (breit) 1H,
austauschbar mit D₂O
6,81 (s) 1H
7,11 (d) 1H
7,68 (s,breit) 1H
7,87 (dd) 1H
8,17 (d) 1H
9,00 (s) 1H,
austauschbar mit D₂O
9,2 (breit) 1H,
austauschbar mit D₂O ppm.

## Patentansprüche (Patentansprüche für folgende(n) Vertragsstaat(en): AT, BE, CH, DE, FR, GB, IT, LI, LU, NL, SE)

1. 6-Oxo-pyridazinderivate der allgemeinen Formel (I) 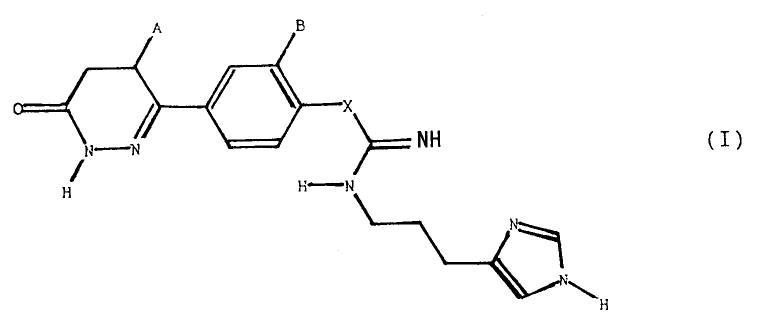 in der A für ein Wasserstoffatom, eine C₁-C₃-Alkylgruppe oder eine Hydroxymethylgruppe steht,
B ein Wasserstoffatom, ein Halogenatom, eine Cyanogruppe oder eine Nitrogruppe ist,
X die Gruppe 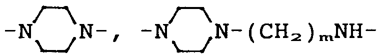 oder 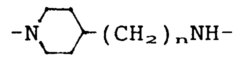 bedeutet, worin m den Wert 2, 3, 4, 5 oder 6 und n den Wert 0, 1, 2, 3 oder 4 haben,
sowie die physiologisch annehmbaren Salze davon.

2. 6-Oxo-pyridazinderivate nach Anspruch 1, dadurch **gekennzeichnet,** daß X die Gruppe 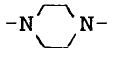 ist.

3. 6-Oxo-pyridazinderivate nach Anspruch 1, dadurch **gekennzeichnet,** daß X die Gruppe 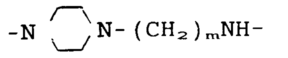 ist, worin m den Wert 2, 3, 4, 5 oder 6 hat.

4. 6-Oxo-pyridazinderivate nach Anspruch 1, dadurch **gekennzeichnet,** daß X die Gruppe 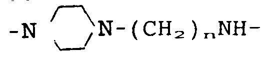 bedeutet, worin n den Wert 0, 1, 2, 3 oder 4 hat.

5. 6-[4-[4-[3-(1H-Imidazol-4-yl)-propylamino-iminomethylen]-piperazin-1-yl]-3-nitro-phenyl]-4,5-dihydro-5-methyl-3(2H)-pyridazinon.

6. N¹-[3-(1H-Imidazol-4-yl)propyl]-N³-[3-[4-[4-(4-methyl-6-oxo-1,4,5,6-tetrahydropyridazin-3-yl)-2-nitro-phenyl]-piperazin-1-yl]propyl]-guanidin.

7. N¹-[3-(1H-Imidazol-4-yl)propyl]-N³-[2-[4-(4-methyl-6-oxo-1,4,5,6-tetrahydropyridazin-3-yl)-2-nitro-anilino]-ethyl]-guanidin.

8. Verfahren zur Herstellung von 6-Oxo-pyridazinderivaten nach den Ansprüchen 1 bis 7 sowie der physiologisch annehmbaren Salze davon, dadurch **gekennzeichnet,** daß man
a) eine Verbindung der allgemeinen Formel (II) 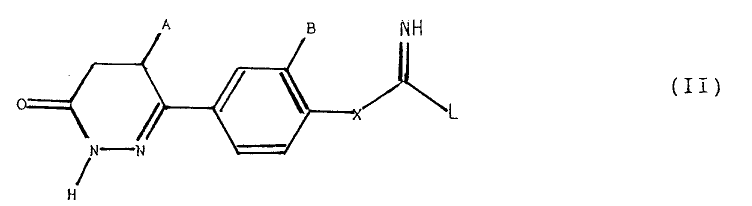 in der A, B und X wie in Anspruch 1 definiert sind und L für eine C₁-C₄-Alkylthio-, eine Phenylthio-, eine C₁-C₄-Alkoxy- oder eine Phenoxygruppe steht,
mit einer Verbindung der Formel (III) 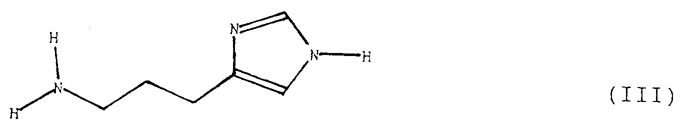 zu einer Verbindung der allgemeinen Formel (I) umsetzt oder
b) eine Verbindung der allgemeinen Formel (IV) 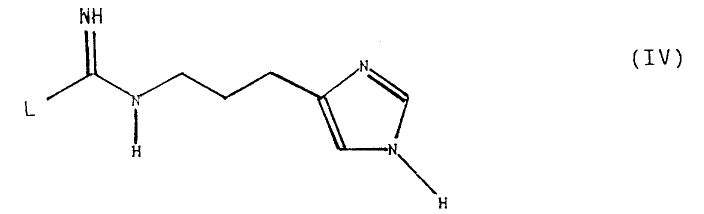 in der L die oben angegebene Bedeutung besitzt,
mit einer Verbindung der allgemeinen Formel (V) 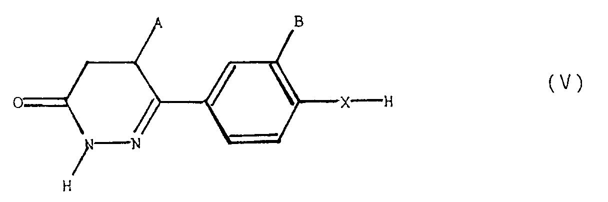 in der A, B und X wie oben definiert sind,
zu einer Verbindung der allgemeinen Formel (I) umsetzt oder
c) eine Verbindung der allgemeinen Formel (Ia) 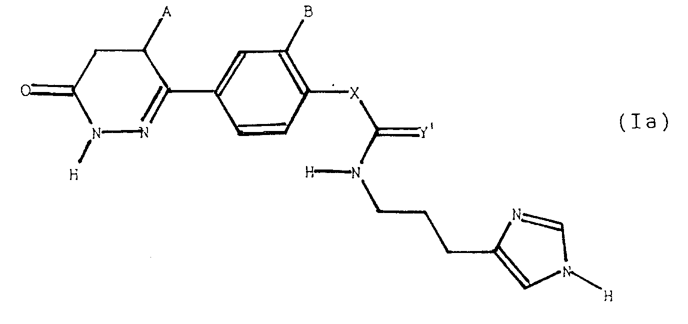 in der A, B und X die oben angegebenen Bedeutungen haben und Y' für eine der Gruppen =N-CN, 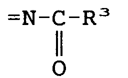 oder 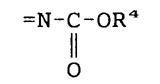 steht, worin R³ für eine gerad- oder verzweigtkettige C₁-C₆-Alkylgruppe oder eine gegebenenfalls mit einem oder mehreren Halogenatomen, C₁-C₃-Alkylgruppen oder C₁-C₃-Alkoxygruppen substituierte Phenyl- oder Naphthylgruppe steht und R⁴ für eine gegebenenfalls mit einem oder mehreren Halogenatomen, C₁-C₃-Alkoxygruppen oder Phenylresten substituierte gerad- oder verzweigtkettige C₁-C₄-Alkylgruppe steht,
sauer oder basisch zu einer Verbindung der allgemeinen Formel (I) hydrolysiert und gegebenenfalls die nach den Verfahrensvarianten a) bis c) erhaltene Verbindung der allgemeinen Formel (I) in an sich bekannter Weise in ihr physiologisch annehmbares Salz umwandelt.

9. Arzneimittel, dadurch **gekennzeichnet,** daß es eine Verbindung nach einem der Ansprüche 1 bis 7 zusammen mit mindestens einem inerten, pharmazeutisch annehmbaren Träger oder Verdünnungsmittel enthält.

## Patentansprüche (Patentansprüche für folgende(n) Vertragsstaat(en): ES)

1. Verfahren zur Herstellung von 6-Oxo-pyridazinderivate der allgemeinen Formel (I) 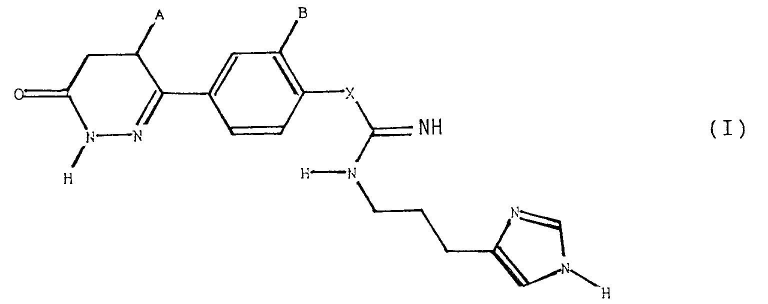 in der A für ein Wasserstoffatom, eine C₁-C₃-Alkylgruppe oder eine Hydroxymethylgruppe steht,
B ein Wasserstoffatom, ein Halogenatom, eine Cyanogruppe oder eine Nitrogruppe ist,
X die Gruppe 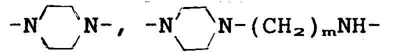 oder 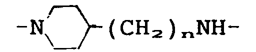 bedeutet, worin m den Wert 2, 3, 4, 5 oder 6 und n den Wert 0, 1, 2, 3 oder 4 haben,
sowie der physiologisch annehmbaren Salze davon, dadurch **gekennzeichnet,** daß man
a) eine Verbindung der allgemeinen Formel (II) 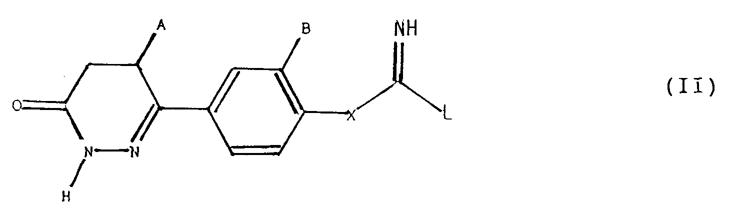 in der A, B und X wie oben definiert sind und L für eine C₁-C₄-Alkylthio-, eine Phenylthio-, eine C₁-C₄-Alkoxy- oder eine Phenoxygruppe steht,
mit einer Verbindung der Formel (III) 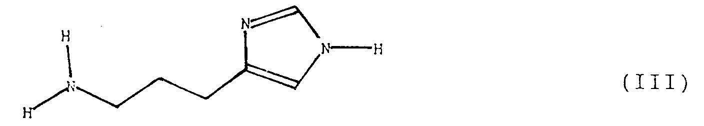 zu einer Verbindung der allgemeinen Formel (I) umsetzt oder
b) eine Verbindung der allgemeinen Formel (IV) 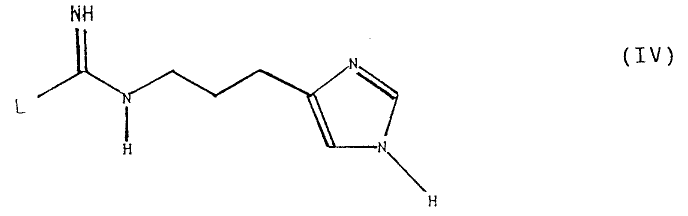 in der L die oben angegebene Bedeutung besitzt,
mit einer Verbindung der allgemeinen Formel (V) 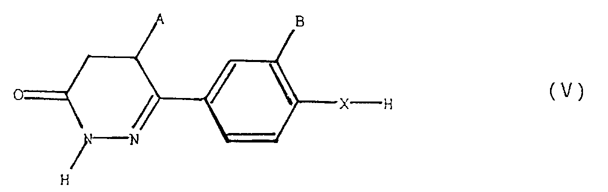 in der A, B und X wie oben definiert sind,
zu einer Verbindung der allgemeinen Formel (I) umsetzt oder
c) eine Verbindung der allgemeinen Formel (Ia) 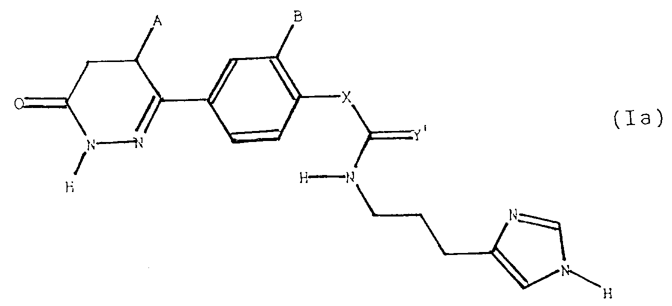 in der A, B und X die oben angegebenen Bedeutungen haben und Y' für eine der Gruppen =N-CN, 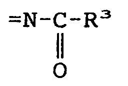 oder 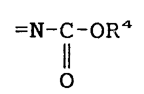 steht, worin R³ für eine gerad- oder verzweigtkettige C₁-C₆-Alkylgruppe oder eine gegebenenfalls mit einem oder mehreren Halogenatomen, C₁-C₃-Alkylgruppen oder C₁-C₃-Alkoxygruppen substituierte Phenyl- oder Naphthylgruppe steht und R⁴ für eine gegebenenfalls mit einem oder mehreren Halogenatomen, C₁-C₃-Alkoxygruppen oder Phenylresten substituierte gerad- oder verzweigtkettige C₁-C₄-Alkylgruppe steht,
sauer oder basisch zu einer Verbindung der allgemeinen Formel (I) hydrolysiert und gegebenenfalls die nach den Verfahrensvarianten a) bis c) erhaltene Verbindung der allgemeinen Formel (I) in an sich bekannter Weise in ihr physiologisch annehmbares Salz umwandelt.

2. Verfahren nach Anspruch 1 zur Herstellung von Verbindungen, bei denen X die Gruppe 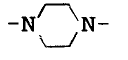 ist.

3. Verfahren nach Anspruch 1 zur Herstellung von Verbindungen, bei denen X die Gruppe 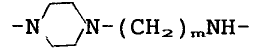 ist, worin m den Wert 2, 3, 4, 5 oder 6 hat.

4. Verfahren nach Anspruch 1 zur Herstellung von Verbindungen, bei denen X die Gruppe 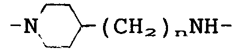 bedeutet, worin n den Wert 0, 1, 2, 3 oder 4 hat.

5. Verfahren nach Anspruch 1 zur Herstellung von 6-[4-[4-[3-(1H-Imidazol-4-yl)-propylamino-iminomethylen]-piperazin- 1-yl]-3-nitro-phenyl]-4,5-dihydro-5-methyl-3(2H)-pyridazinon.

6. Verfahren nach Anspruch 1 zur Herstellung von N¹-[3-(1H-Imidazol-4-yl)propyl]-N³-[3-[4-[4-(4-methyl-6-oxo-1,4,5,6-tetrahydropyridazin-3-yl)-2-nitro-phenyl]-piperazin-1-yl]propyl]-guanidin.

7. Verfahren nach Anspruch 1 zur Herstellung von N¹-[3-(1H-Imidazol-4-yl)propyl]-N³-[2-[4-(4-methyl-6-oxo-1,4,5,6-tetrahydropyridazin-3-yl)-2-nitro-anilino]-ethyl]-guanidin.

## Patentansprüche (Patentansprüche für folgende(n) Vertragsstaat(en): GR)

1. 6-Oxo-pyridazinderivate der allgemeinen Formel (I) 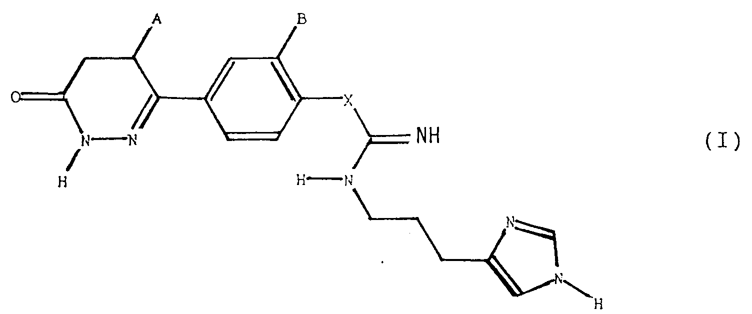 in der A für ein Wasserstoffatom, eine C₁-C₃-Alkylgruppe oder eine Hydroxymethylgruppe steht,
B ein Wasserstoffatom, ein Halogenatom, eine Cyanogruppe oder eine Nitrogruppe ist,
X die Gruppe 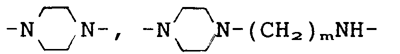 oder 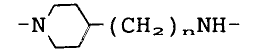 bedeutet, worin m den Wert 2, 3, 4, 5 oder 6 und n den Wert 0, 1, 2, 3 oder 4 haben,
sowie die Salze davon.

2. 6-Oxo-pyridazinderivate nach Anspruch 1, dadurch **gekennzeichnet,** daß X die Gruppe 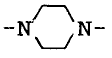 ist.

3. 6-Oxo-pyridazinderivate nach Anspruch 1, dadurch **gekennzeichnet,** daß X die Gruppe 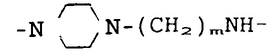 ist, worin m den Wert 2, 3, 4, 5 oder 6 hat.

4. 6-Oxo-pyridazinderivate nach Anspruch 1, dadurch **gekennzeichnet,** daß X die Gruppe 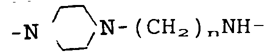 bedeutet, worin n den Wert 0, 1, 2, 3 oder 4 hat.

5. 6-[4-[4-[3-(1H-Imidazol-4-yl)-propylamino-iminomethylen]-piperazin-1-yl]-3-nitro-phenyl]-4,5-dihydro-5-methyl-3(2H)-pyridazinon.

6. N¹-[3-(1H-Imidazol-4-yl)propyl]-N³-[3-[4-[4-(4-methyl-6-oxo-1,4,5,6-tetrahydropyridazin-3-yl)-2-nitro-phenyl]-piperazin-1-yl]propyl]-guanidin.

7. N¹-[3-(1H-Imidazol-4-yl)propyl]-N³-[2-[4-(4-methyl-6-oxo-1,4,5,6-tetrahydropyridazin-3-yl)-2-nitro-anilino]-ethyl]-guanidin.

8. Verfahren zur Herstellung von 6-Oxo-pyridazinderivaten nach den Ansprüchen 1 bis 7 sowie der Salze davon, dadurch **gekennzeichnet,** daß man
a) eine Verbindung der allgemeinen Formel (II) 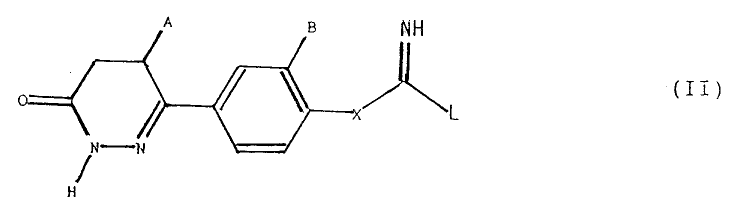 in der A, B und X wie in Anspruch 1 definiert sind und L für eine C₁-C₄-Alkylthio-, eine Phenylthio-, eine C₁-C₄-Alkoxy- oder eine Phenoxygruppe steht,
mit einer Verbindung der Formel (III) 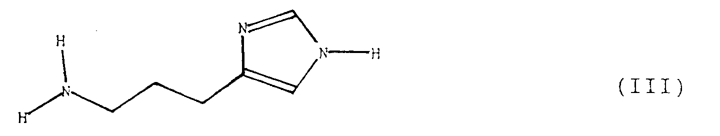 zu einer Verbindung der allgemeinen Formel (I) umsetzt oder
b) eine Verbindung der allgemeinen Formel (IV) 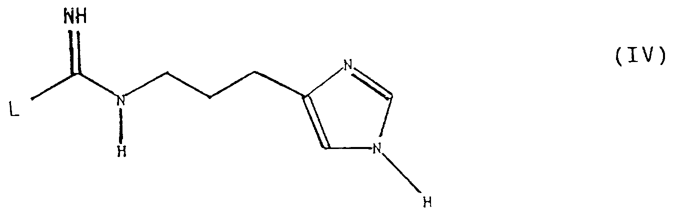 in der L die oben angegebene Bedeutung besitzt,
mit einer Verbindung der allgemeinen Formel (V) 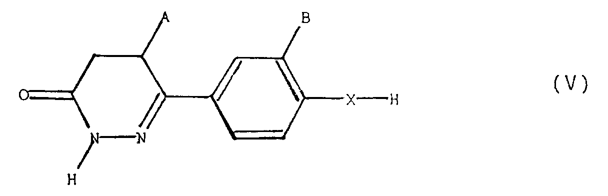 in der A, B und X wie oben definiert sind,
zu einer Verbindung der allgemeinen Formel (I) umsetzt oder
c) eine Verbindung der allgemeinen Formel (Ia) 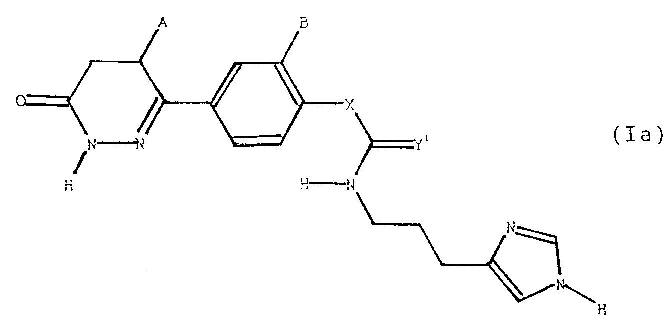 in der A, B und X die oben angegebenen Bedeutungen haben und Y' für eine der Gruppen =N-CN, 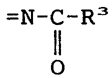 oder 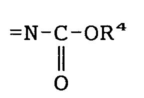 steht, worin R³ für eine gerad- oder verzweigtkettige C₁-C₆-Alkylgruppe oder eine gegebenenfalls mit einem oder mehreren Halogenatomen, C₁-C₃-Alkylgruppen oder C₁-C₃-Alkoxygruppen substituierte Phenyl- oder Naphthylgruppe steht und R⁴ für eine gegebenenfalls mit einem oder mehreren Halogenatomen, C₁-C₃-Alkoxygruppen oder Phenylresten substituierte gerad- oder verzweigtkettige C₁-C₄-Alkylgruppe steht,
sauer oder basisch zu einer Verbindung der allgemeinen Formel (I) hydrolysiert und gegebenenfalls die nach den Verfahrensvarianten a) bis c) erhaltene Verbindung der allgemeinen Formel (I) in an sich bekannter Weise in ihr Salz umwandelt.

## Claims (Claims for the following Contracting State(s): AT, BE, CH, DE, FR, GB, IT, LI, LU, NL, SE)

1. 6-oxopyridazine derivatives of the general formula (I) 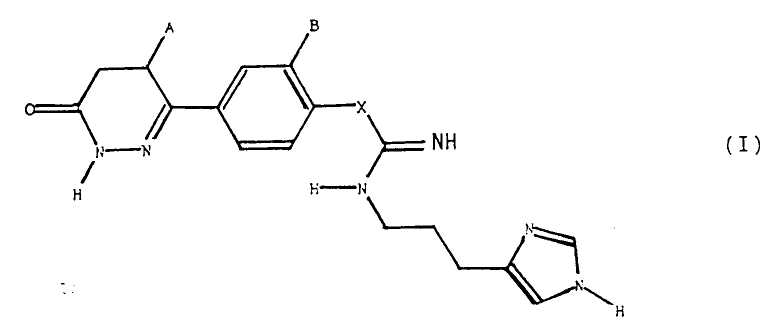 wherein A represents a hydrogen atom, a C₁ - C₃ alkyl group or a hydroxymethyl group,
B is a hydrogen atom, a halogen atom, a cyano group or a nitro group,
X signifies the groups 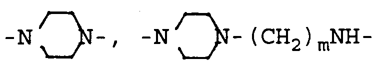 or 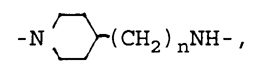 wherein m has the value 2, 3, 4, 5 or 6 and n has the value 0, 1, 2, 3 or 4,
and the physiologically acceptable salts thereof.

2. 6-oxopyridazine derivatives according to claim 1, characterised in that X signifies the group

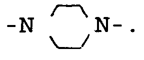

3. 6-oxopyridazine derivatives according to claim 1, characterised in that X signifies the group 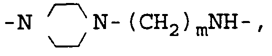 wherein m has the value 2, 3, 4, 5 or 6.

4. 6-oxopyridazine derivatives according to claim 1, characterised in that X signifies the group 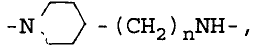 wherein n has the value 0, 1, 2, 3, or 4.

5. 6-[4-[4-[3-(1H-imidazol-4-yl) propylaminoiminomethylene]-piperazin-1-yl]-3-nitrophenyl]-4,5-dihydro-5-methyl-3(2H)pyridazinone.

6. N¹-[3-(1H-imidazol-4-yl)propyl]-N³-[3-[4-[4-(4-methyl-6-oxo-1,4,5,6-tetrahydropyridazin-3-yl)-2-nitrophenyl]-piperazin-1-yl]propyl]guanidine.

7. N¹-[3-(1H-imidazol-4-yl)propyl]-N³-[2-[4-(4-methyl-6-oxo-1,4,5,6-tetrahydropyridazin-3-yl)-2-nitroanilino]-ethyl]guanidine.

8. Method for the preparation of 6-oxopyridazine derivatives according to claims 1 to 7 and the physiologically acceptable salts thereof, characterised in that
a) a compound of the general formula (II) 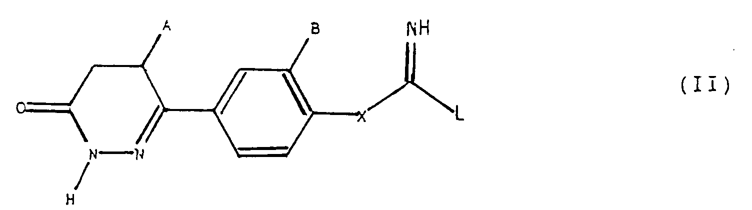 wherein A, B and X are defined as in claim 1 and L represents a C₁ - C₄ alkylthio group, a phenylthio group, a C₁ - C₄ alkoxy group or a phenoxy group,
is reacted with a compound of the formula (III) 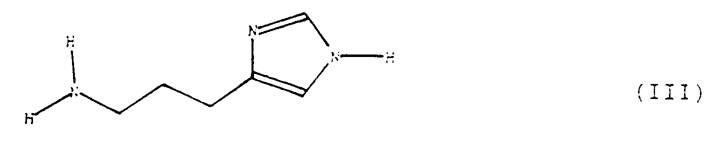 to form a compound of the general formula (I) or
b) a compound of the general formula (IV) 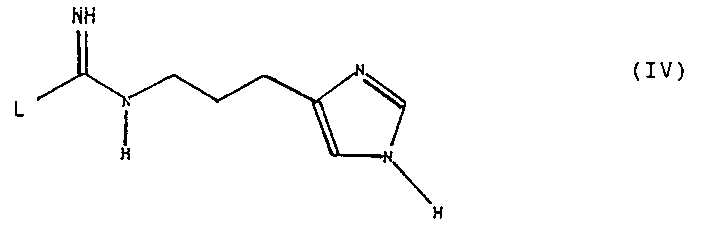 wherein L has the meaning given above,
is reacted with a compound of the general formula (V) 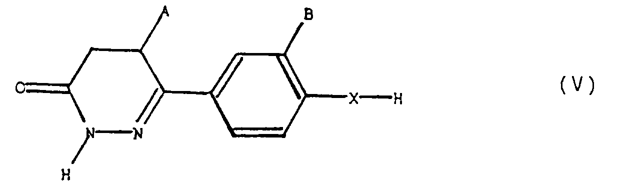 wherein A, B and X are defined as above,
to form a compound of the general formula (I) or
c) a compound of the general formula (Ia) 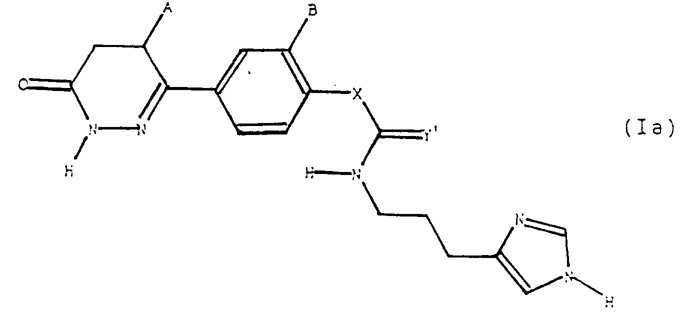 wherein A, B and X have the meanings given above and Y' represents one of the groups
=N-CN, 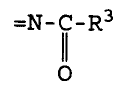 or 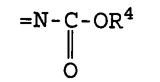 wherein R³ represents a straight-chain or branched-chain C₁ - C₆ alkyl group or a phenyl group or naphthyl group optionally substituted by one or more halogen atoms, C₁ - C₃ alkyl groups or C₁ - C₃ alkoxy groups and R⁴ represents a straight-chain or branched-chain C₁ - C₄ alkyl group optionally substituted by one or more halogen atoms, C₁ - C₃ alkoxy groups or phenyl radicals,
is hydrolysed acidically or basically to form a compound of the general formula (I) and optionally the compound of the general formula (I) obtained by the variants a) to c) of the method is converted in a manner known per se to its physiologically acceptable salt.

9. Medicine, characterised in that it contains a compound according to any one of claims 1 to 7 together with at least one inert, pharmaceutically acceptable carrier or diluent.

## Claims (Claims for the following Contracting State(s): ES)

1. Method for the preparation of 6-oxopyridazine derivatives of the general formula (I) 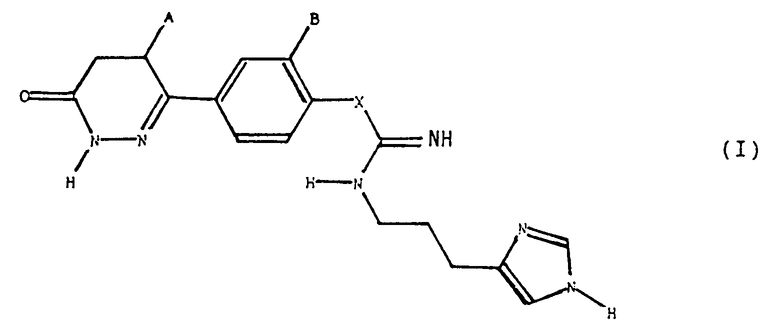 wherein A represents a hydrogen atom, a C₁ - C₃ alkyl group or a hydroxymethyl group,
B is a hydrogen atom, a halogen atom, a cyano group or a nitro group,
X signifies the groups 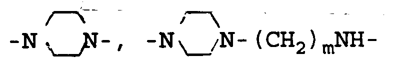 or 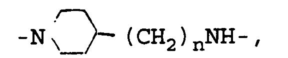 wherein m has the value 2, 3, 4, 5 or 6 and n has the value 0, 1, 2, 3 or 4,
and the physiologically acceptable salts thereof,
characterised in that
a) a compound of the general formula (II) 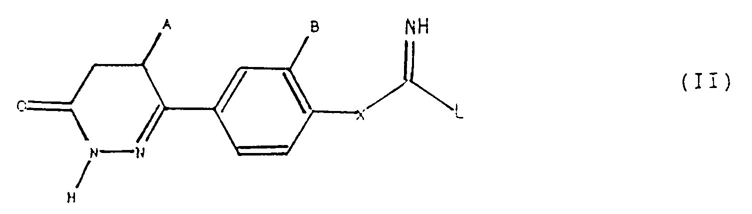 wherein A, B and X are defined as in claim 1 and L represents a C₁ - C₄ alkylthio group, a phenylthio group, a C₁ - C₄ alkoxy group or a phenoxy group,
is reacted with a compound of the formula (III) 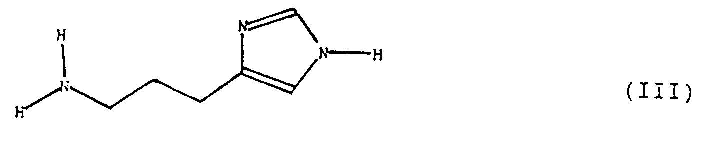 to form a compound of the general formula (I) or
b) a compound of the general formula (IV) 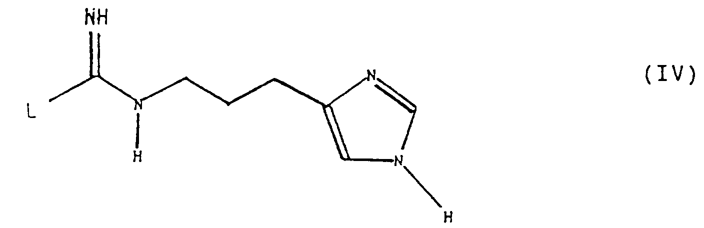 wherein L has the meaning given above,
is reacted with a compound of the general formula (V) 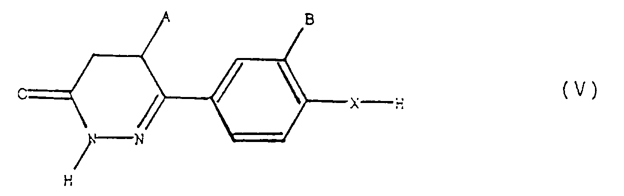 wherein A, B and X are defined as above,
to form a compound of the general formula (I) or
c) a compound of the general formula (Ia) 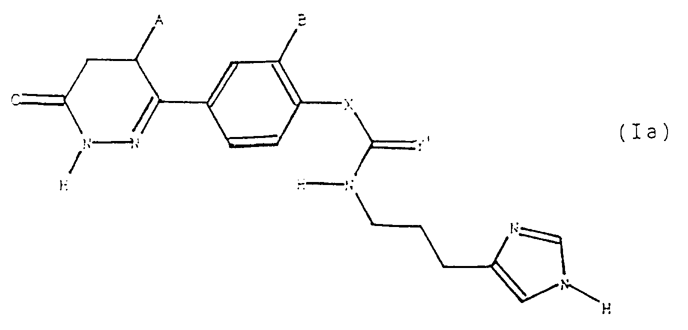 wherein A, B and X have the meanings given above and Y' represents one of the groups
=N-CN, 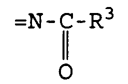 or 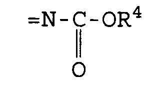 wherein R³ represents a straight-chain or branched-chain C₁ - C₆ alkyl group or a phenyl group or naphthyl group optionally substituted by one or more halogen atoms, C₁ - C₃ alkyl groups or C₁ - C₃ alkoxy groups and R⁴ represents a straight-chain or branched-chain C₁ - C₄ alkyl group optionally substituted by one or more halogen atoms, C₁ - C₃ alkoxy groups or phenyl radicals,
is hydrolysed acidically or basically to form a compound of the general formula (I) and optionally the compound of the general formula (I) obtained by the variants a) to c) of the method is converted in a manner known per se to its physiologically acceptable salt.

2. Method according to claim 1 for the preparation of compounds, wherein X signifies the group

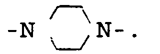

3. Method according to claim 1 for the preparation of compounds, wherein X signifies the group 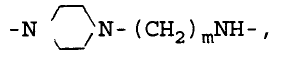 wherein m has the value 2, 3, 4, 5 or 6.

4. Method according to claim 1 for the preparation of compounds, wherein X signifies the group 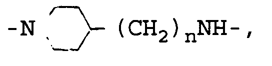 wherein n has the value 0, 1, 2, 3 or 4.

5. Method according to claim 1 for the preparation of 6-[4-[4-[3-(1H-imidazol-4-yl)propylaminoiminomethylene] -piperazin-1-yl]-3-nitrophenyl]-4,5-dihydro-5-methyl-3(2H)pyridazinone.

6. Method according to claim 1 for the preparation of N¹-[3-(1H-imidazol-4-yl)propyl]-N³-[3-[4-[4-(4-methyl-6-oxo-1,4,5,6-tetrahydropyridazin-3-yl)-2-nitrophenyl]-piperazin-1-yl]propyl]guanidine.

7. Method according to claim 1 for the preparation of N¹-[3-(1H-imidazol-4-yl)propyl]-N³-[2-[4-[4-(4-methyl-6-oxo-1,4,5,6-tetrahydropyridazin-3-yl)-2-nitroanilino]-ethyl]guanidine.

## Claims (Claims for the following Contracting State(s): GR)

1. 6-oxopyridazine derivatives of the general formula (I) 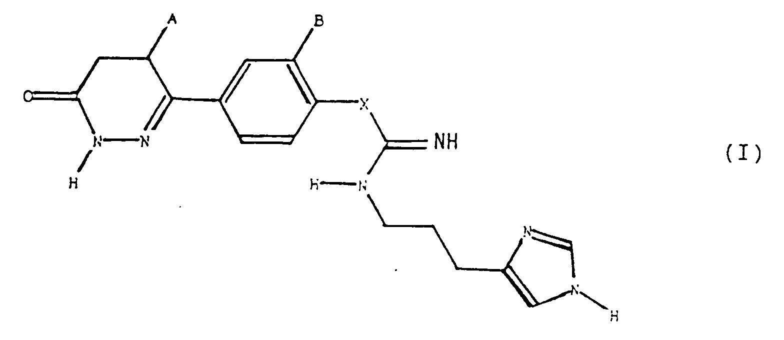 wherein A represents a hydrogen atom, a C₁ - C₃ alkyl group or a hydroxymethyl group,
B is a hydrogen atom, a halogen atom, a cyano group or a nitro group,
X signifies the groups 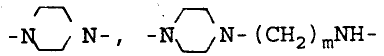 or 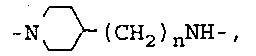 wherein m has the value 2, 3, 4, 5 or 6 and n has the value 0, 1, 2, 3 or 4,
and the salts thereof.

2. 6-oxopyridazine derivatives according to claim 1, characterised in that X signifies the group

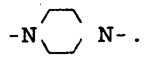

3. 6-oxopyridazine derivatives according to claim 1, characterised in that X signifies the group 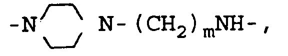 wherein m has the value 2, 3, 4, 5 or 6.

4. 6-oxopyridazine derivatives according to claim 1, characterised in that X signifies the group 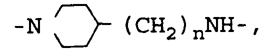 wherein n has the value 0, 1, 2, 3, or 4.

5. 6-[4-[4-[3-(1H-imidazol-4-yl) propylaminoiminomethylene]-piperazin-1-yl]-3-nitrophenyl]-4,5-dihydro-5-methyl-3(2H)pyridazinone.

6. N¹-[3-(1H-imidazol-4-yl)propyl]-N³-[3-[4-[4-(4-methyl-6-oxo-1,4,5,6-tetrahydropyridazin-3-yl)-2-nitrophenyl]-piperazin-1-yl]propyl]guanidine.

7. N¹-[3-(1H-imidazol-4-yl)propyl]-N³-[2-[4-(4-methyl-6-oxo-1,4,5,6-tetrahydropyridazin-3-yl)-2-nitroanilino]-ethyl]guanidine.

8. Method for the preparation of 6-oxopyridazine derivatives according to claims 1 to 7 and the salts thereof, characterised in that
a) a compound of the general formula (II) 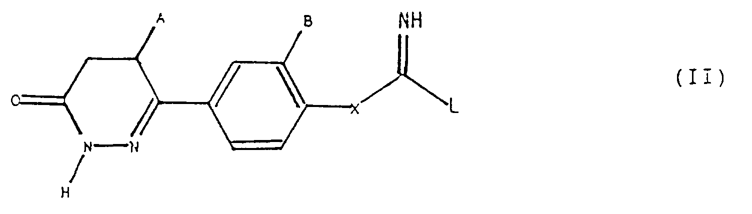 wherein A, B and X are defined as in claim 1 and L represents a C₁ - C₄ alkylthio group, a phenylthio group, a C₁ - C₄ alkoxy group or a phenoxy group,
is reacted with a compound of the formula (III) 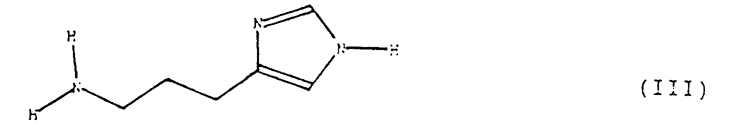 to form a compound of the general formula (I) or
b) a compound of the general formula (IV) 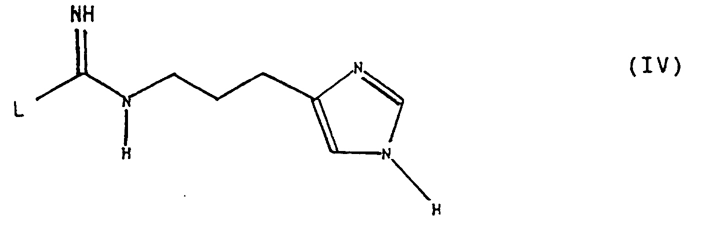 wherein L has the meaning given above,
is reacted with a compound of the general formula (V) 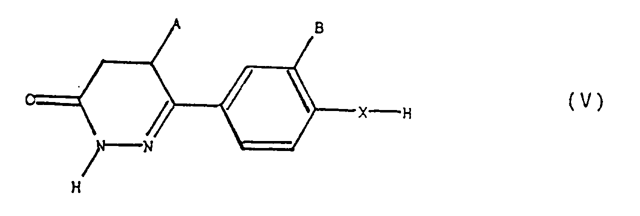 wherein A, B and X are defined as above,
to form a compound of the general formula (I) or
c) a compound of the general formula (Ia) 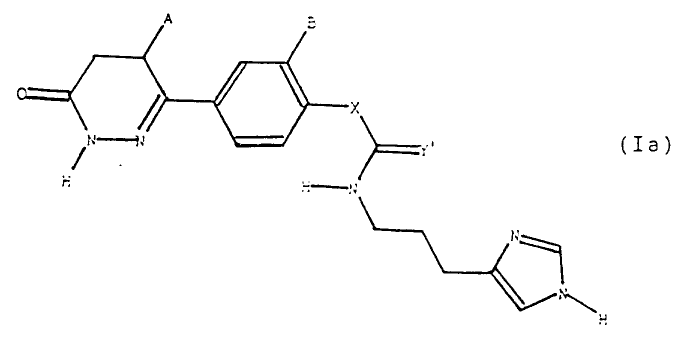 wherein A, B and X have the meanings given above and Y' represents one of the groups
=N-CN, 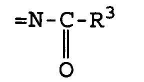 or 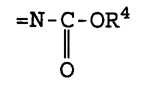 wherein R³ represents a straight-chain or branched-chain C₁ - C₆ alkyl group or a phenyl group or naphthyl group optionally substituted by one or more halogen atoms, C₁ - C₃ alkyl groups or C₁ - C₃ alkoxy groups and R⁴ represents a straight-chain or branched-chain C₁ - C₄ alkyl group optionally substituted by one or more halogen atoms, C₁ - C₃ alkoxy groups or phenyl radicals,
is hydrolysed acidically or basically to form a compound of the general formula (I) and optionally the compound of the general formula (I) obtained by the variants a) to c) of the method is converted in a manner known per se to its salt.

## Revendications (Revendications pour l'(les) Etat(s) contractant(s) suivant(s): AT, BE, CH, DE, FR, GB, IT, LI, LU, NL, SE)

1. Dérivés de 6-oxo-pyridazine répondant à la formule générale (I) : 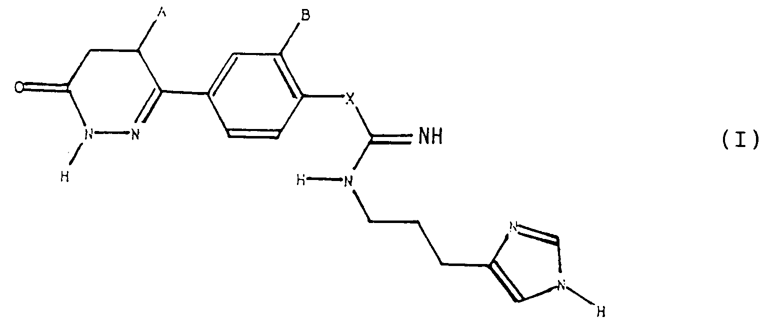 où A représente un atome d'hydrogène, un groupe alkyle en C₁-C₃ ou un groupe hydroxyméthyle,
B représente un atome d'hydrogène, un atome d'halogène, un groupe cyano ou un groupe nitro,
X représente le groupe 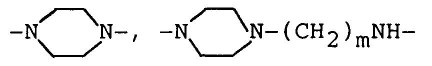 ou 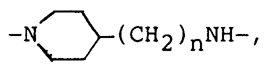 où m a la valeur de 2, 3, 4, 5 ou 6, et n a la valeur de 0, 1, 2, 3 ou 4,
ainsi que leurs sels physiologiquement acceptables.

2. Dérivés de 6-oxo-pyridazine selon la revendication 1, caractérisés en ce que X est le groupe

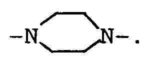

3. Dérivés de 6-oxo-pyridazine selon la revendication 1, caractérisés en ce que X est le groupe 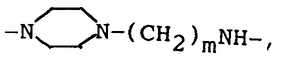 où m a la valeur de 2, 3, 4, 5 ou 6.

4. Dérivés de 6-oxo-pyridazine selon la revendication 1, caractérisés en ce que X est le groupe 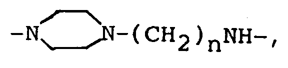 où n a la valeur de 0, 1, 2, 3 ou 4.

5. 6-[4-[4-[3-(1H-imidazol-4-yl)-propylamino-iminométhylène]-pipérazine-1-yl]-3-nitro-phényl]-4,5-dihydro- 5-méthyl-3(2H)-pyridazinone.

6. N¹-[3-(1H-imidazol-4-yl)propyl]-N³-[3-[4-[4-(4-méthyl-6-oxo-1,4,5,6-tétrahydropyridazine-3-yl)-2-nitro-phényl]-pipérazine-1-yl]propyl]-guanidine.

7. N¹-[3-(1H-imidazol-4-yl)propyl]-N³-[2-[4-(4-méthyl-6-oxo-1,4,5,6-tétrahydropyridazine-3-yl)-2-nitro-anilino]-éthyl]-guanidine.

8. Procédé de préparation de dérivés de 6-oxo-pyridazine selon les revendications 1 à 7, ainsi que de leurs sels physiologiquement acceptables, caractérisé en ce que :
a) on fait réagir un composé de formule générale (II) 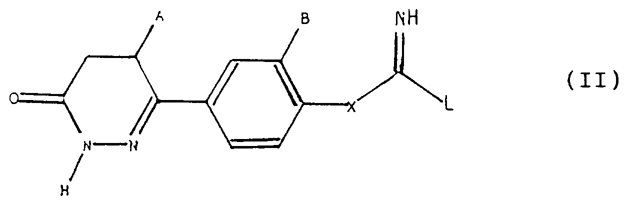 où A, B et X sont définis comme dans la revendication 1, et L représente un groupe alkylthio en C₁-C₄, un groupe phénylthio, un groupe alcoxy en C₁-C₄ ou un groupe phénoxy,
avec un composé de formule (III) 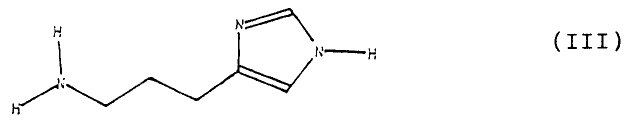 pour obtenir un composé de formule (I), ou
b) on fait réagir un composé de formule générale (IV) 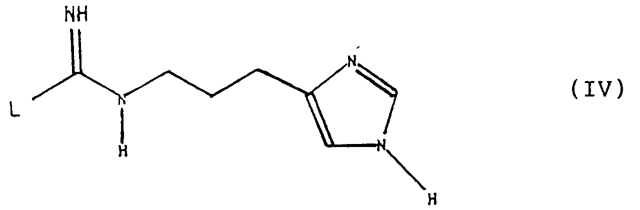 où L a la signification indiquée ci-dessus,
avec un composé de formule générale (V) 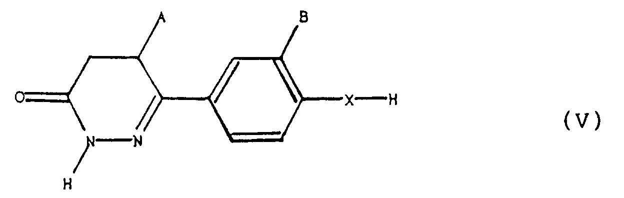 où A, B et X sont définis comme ci-dessus,
pour obtenir un composé de formule générale (I), ou
c) on hydrolyse un composé de formule générale (Ia) 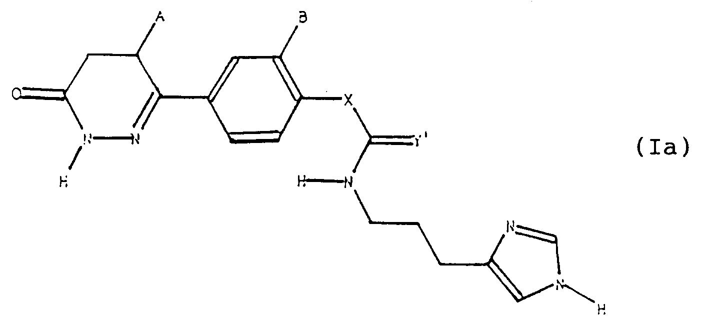 où A, B et X ont les significations indiquées ci-dessus, et Y' représente un des groupes =N-CN, 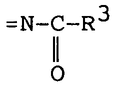 ou 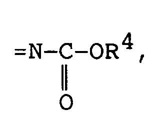 où R³ représente un groupe alkyle en C₁-C₆ à chaîne droite ou ramifiée ou un groupe phényle ou naphtyle éventuellement substitué par un ou plusieurs atomes d'halogène, par des groupes alkyles en C₁-C₃ ou par des groupes alcoxy en C₁-C₃, et R⁴ représente un groupe alkyle en C₁-C₄ à chaîne droite ou ramifiée, éventuellement substitué par un ou plusieurs atomes d'halogène, par des groupes alcoxy en C₁-C₃ ou par des restes phényle,
en milieu acide ou basique, pour obtenir un composé de formule générale (I) et éventuellement, on transforme le composé de formule générale (I), obtenu d'après les variantes de procédé a) à c), d'une manière connue en soi, en son sel physiologiquement acceptable.

9. Médicament caractérisé en ce qu'il contient un composé selon l'une des revendications 1 à 7 conjointement avec au moins un véhicule ou un diluant inerte pharmaceutiquement acceptable.

## Revendications (Revendications pour l'(les) Etat(s) contractant(s) suivant(s): ES)

1. Procédé de préparation de dérivés de 6-oxo-pyridazine répondant à la formule générale (I) 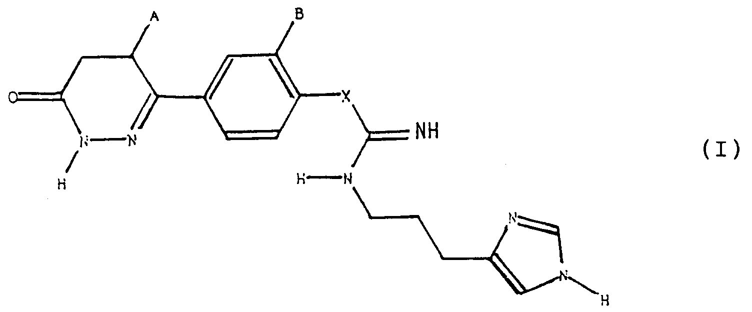 où A représente un atome d'hydrogène, un groupe alkyle en C₁-C₃ ou un groupe hydroxyméthyle,
B représente un atome d'hydrogène, un atome d'halogène, un groupe cyano ou un groupe nitro,
X représente le groupe 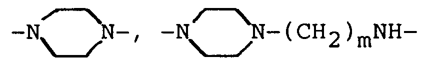 ou 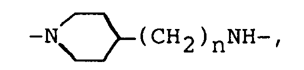 où m a la valeur de 2, 3, 4, 5 ou 6, et n a la valeur de 0, 1, 2, 3 ou 4,
ainsi que leurs sels physiologiquement acceptables,
caractérisé en ce que :
a) on fait réagir un composé de formule générale (II) 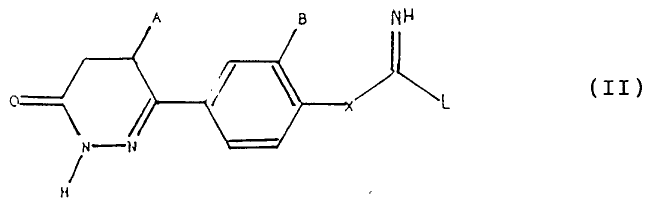 où A, B et X sont définis comme ci-dessus, et L représente un groupe alkylthio en C₁-C₄, un groupe phénylthio, un groupe alcoxy en C₁-C₄ ou un groupe phénoxy,
avec un composé de formule (III) 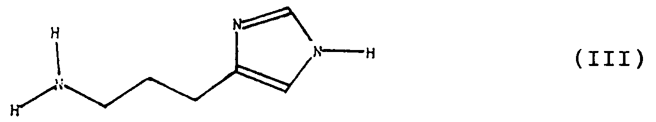 pour obtenir un composé de formule (I), ou
b) on fait réagir un composé de formule générale (IV) 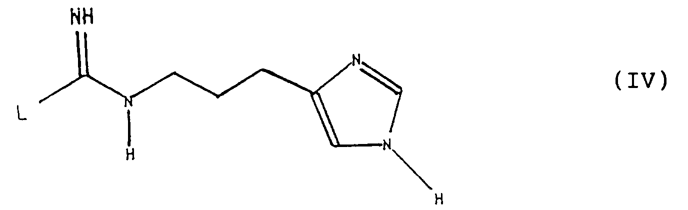 où L a la signification indiquée ci-dessus,
avec un composé de formule générale (V) 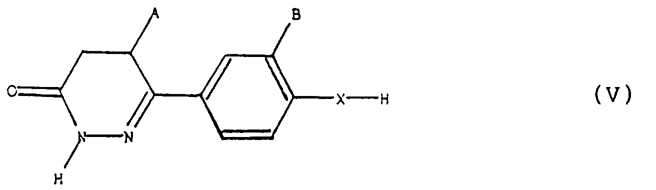 où A, B et X sont définis comme ci-dessus,
pour obtenir un composé de formule générale (I), ou
c) on hydrolyse un composé de formule générale (Ia) 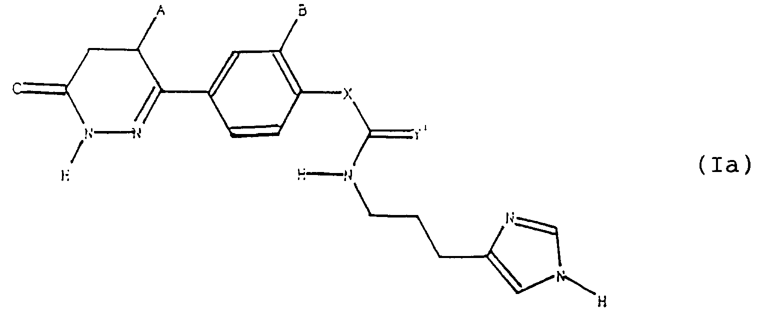 où A, B et X ont les significations indiquées ci-dessus, et Y' représente un des groupes =N-CN, 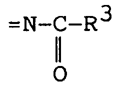 ou 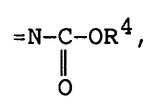 où R³ représente un groupe alkyle en C₁-C₆ à chaîne droite ou ramifiée ou un groupe phényle ou naphtyle éventuellement substitué par un ou plusieurs atomes d'halogène, par des groupes alkyles en C₁-C₃ ou par des groupes alcoxy en C₁-C₃, et R⁴ représente un groupe alkyle en C₁-C₄ à chaîne droite ou ramifiée, éventuellement substitué par un ou plusieurs atomes d'halogène, par des groupes alcoxy en C₁-C₃ ou par des restes phényle,
en milieu acide ou basique, pour obtenir un composé de formule générale (I) et éventuellement, on transforme le composé de formule générale (I), obtenu d'après les variantes de procédé a) à c), d'une manière connue en soi, en son sel physiologiquement acceptable.

2. Procédé selon la revendication 1 pour la préparation de composés dans lesquels X est le groupe

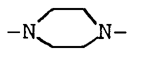

3. Procédé selon la revendication 1 pour la préparation de composés dans lesquels X est le groupe 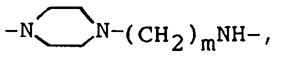 où m a la valeur de 2, 3, 4, 5 ou 6.

4. Procédé selon la revendication 1 pour la préparation de composés dans lesquels X est le groupe 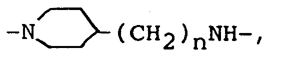 où n a la valeur de 0, 1, 2, 3 ou 4.

5. Procédé selon la revendication 1 pour la préparation de 6-[4-[4-[3-(1H-imidazol-4-yl)-propylamino-iminométhylène]-pipérazine-1-yl]-3-nitro-phényl]-4,5-dihydro-5-méthyl-3(2H)-pyridazinone.

6. Procédé selon la revendication 1 pour la préparation de N¹-[3-(1H-imidazol-4-yl)propyl]-N³-[3-[4-[4-(4-méthyl-6-oxo-1,4,5,6-tétrahydropyridazine-3-yl)-2-nitro-phényl]-pipérazine-1-yl]propyl]-guanidine.

7. Procédé selon la revendication 1 pour la préparation de N¹-[3-(1H-imidazol-4-yl)propyl]-N³-[2-[4-(4-méthyl-6-oxo-1,4,5,6-tétrahydropyridazine-3-yl)-2-nitro-anilino]-éthyl]-guanidine.

## Revendications (Revendications pour l'(les) Etat(s) contractant(s) suivant(s): GR)

1. Dérivés de 6-oxo-pyridazine répondant à la formule générale (I) : 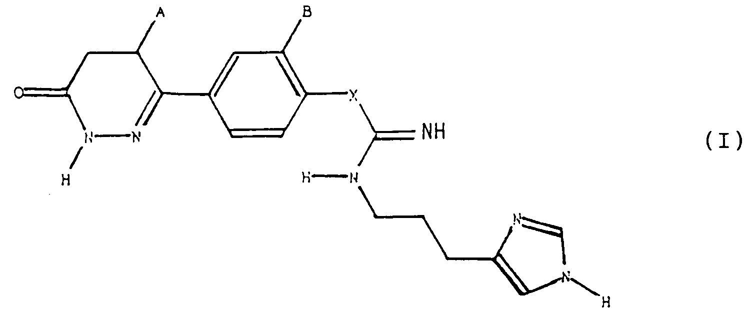 où A représente un atome d'hydrogène, un groupe alkyle en C₁-C₃ ou un groupe hydroxyméthyle,
B représente un atome d'hydrogène, un atome d'halogène, un groupe cyano ou un groupe nitro,
X représente le groupe 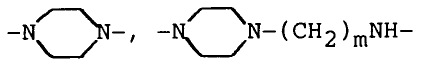 ou 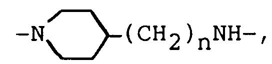 où m a la valeur de 2, 3, 4, 5 ou 6, et n a la valeur de 0, 1, 2, 3 ou 4,
ainsi que leurs sels.

2. Dérivés de 6-oxo-pyridazine selon la revendication 1, caractérisés en ce que X est le groupe

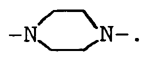

3. Dérivés de 6-oxo-pyridazine selon la revendication 1, caractérisés en ce que X est le groupe 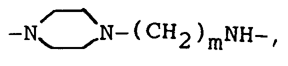 où m a la valeur de 2, 3, 4, 5 ou 6.

4. Dérivés de 6-oxo-pyridazine selon la revendication 1, caractérisés en ce que X est le groupe 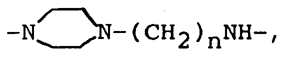 où n a la valeur de 0, 1, 2, 3 ou 4.

5. 6-[4-[4-[3-(1H-imidazol-4-yl)-propylamino-iminométhylène]-pipérazine-1-yl]-3-nitro-phényl]-4,5-dihydro- 5-méthyl-3(2H)-pyridazinone.

6. N¹-[3-(1H-imidazol-4-yl)propyl]-N³-[3-[4-[4-(4-méthyl-6-oxo-1,4,5,6-tétrahydropyridazine-3-yl)-2-nitro-phényl]-pipérazine-1-yl]propyl]-guanidine.

7. N¹-[3-(1H-imidazol-4-yl)propyl]-N³-[2-[4-(4-méthyl-6-oxo-1,4,5,6-tétrahydropyridazine-3-yl)-2-nitro-anilino]-éthyl]-guanidine.

8. Procédé de préparation de dérivés de 6-oxo-pyridazine selon les revendications 1 à 7, ainsi que de leurs sels, caractérisé en ce que :
a) on fait réagir un composé de formule générale (II) 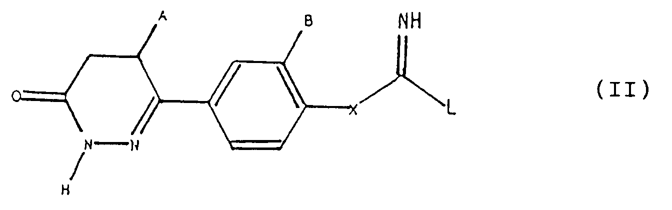 où A, B et X sont définis comme dans la revendication 1 et L représente un groupe alkylthio en C₁-C₄, un groupe phénylthio, un groupe alcoxy en C₁-C₄ ou un groupe phénoxy,
avec un composé de formule (III) 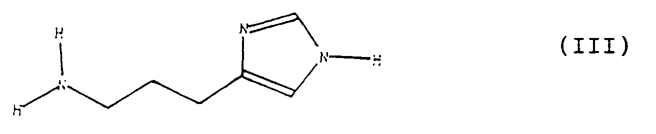 pour obtenir un composé de formule (I), ou
b) on fait réagir un composé de formule générale (IV) 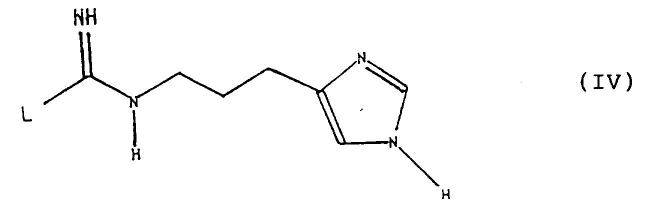 où L a la signification indiquée ci-dessus,
avec un composé de formule générale (V) 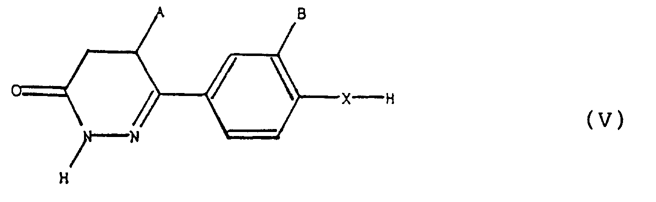 où A, B et X sont définis comme ci-dessus,
pour obtenir un composé de formule générale (I), ou
c) on hydrolyse un composé de formule générale (Ia) 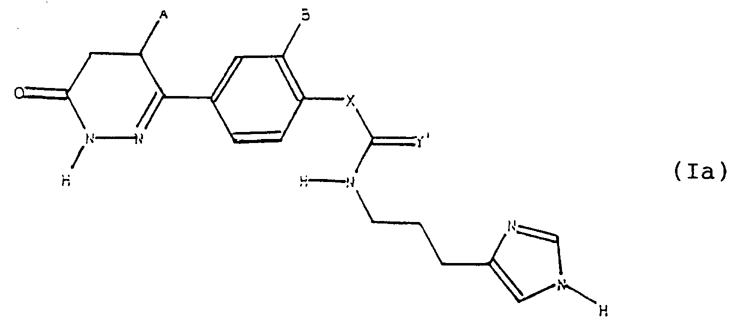 où A, B et X ont les significations indiquées ci-dessus, et Y' représente un des groupes =N-CN, 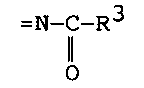 ou 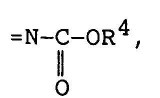 où R³ représente un groupe alkyle en C₁-C₆ à chaîne droite ou ramifiée ou un groupe phényle ou naphtyle éventuellement substitué par un ou plusieurs atomes d'halogène, par des groupes alkyles en C₁-C₃ ou par des groupes alcoxy en C₁-C₃, et R⁴ représente un groupe alkyle en C₁-C₄ à chaîne droite ou ramifiée, éventuellement substitué par un ou plusieurs atomes d'halogène, par des groupes alcoxy en C₁-C₃ ou par des restes phényle,
en milieu acide ou basique, pour obtenir un composé de formule générale (I) et éventuellement, on transforme le composé de formule générale (I), obtenu d'après les variantes de procédé a) à c), d'une manière connue en soi, en son sel.
